(19) Europäisches Patentamt
European Patent Office
Office européen des brevets

(11) Numéro de publication : **0 440 542 B1**

(12) **FASCICULE DE BREVET EUROPEEN**

(45) Date de publication du fascicule du brevet : **12.01.94 Bulletin 94/02**

(51) Int. Cl.$^5$ : **A61K 7/08**, A61K 7/06, A61K 7/48

(21) Numéro de dépôt : **91400191.2**

(22) Date de dépôt : **29.01.91**

# (54) Compositions lavantes à base de silicones insolubles et d'un agent tensio-actif du type acide éther carboxylique polyoxyalkyléné, et leur application en cosmétique et en dermatologie.

Le dossier contient des informations techniques présentées postérieurement au dépôt de la demande et ne figurant pas dans le présent fascicule.

(30) Priorité : **31.01.90 FR 9001149**

(43) Date de publication de la demande : **07.08.91 Bulletin 91/32**

(45) Mention de la délivrance du brevet : **12.01.94 Bulletin 94/02**

(84) Etats contractants désignés : **AT BE CH DE DK ES FR GB GR IT LI NL SE**

(56) Documents cités :
**EP-A- 0 095 238**
**EP-A- 0 331 915**
**EP-A- 0 346 582**

(56) Documents cités :
**SEIFEN, OLE, FETTE, WACHSE. vol. 109, no. 13, 11 août 1983, AUGSBURG DE pages 353 - 355; N.A.I. van Paassen: "Alkylethercarboxylate:Hautfreundliche Rohstoffe für kosmetische Anwendungen"**

(73) Titulaire : **L'OREAL**
**14, Rue Royale**
**F-75008 Paris (FR)**

(72) Inventeur : **Sebag, Henri**
**26, rue Erlanger**
**F-75016 Paris (FR)**
Inventeur : **Dubief, Claude**
**9, rue Edmond Rostand**
**F-78150 Le Chesnay (FR)**

(74) Mandataire : **Casalonga, Axel et al**
**BUREAU D.A. CASALONGA - JOSSE**
**Morassistrasse 8**
**D-80469 München (DE)**

EP 0 440 542 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne des compositions lavantes à base de silicones insolubles et d'un agent tensio-actif du type acide éther carboxylique polyoxyalkyléné ou ses sels, et leur application en cosmétique et en dermatologie.

On utilise déjà des huiles siliconées en cosmétique comme lubrifiants dans des compositions de traitement des cheveux ou de la peau. On connait notamment des compositions de traitement des cheveux ou de la peau à base de polyorganosiloxanes à fonction B-cetoester telles que décrites dans la demande EP 0 331 915.

La plupart des huiles siliconées sont insolubles dans l'eau et difficiles à mettre en oeuvre.

La demanderesse vient de découvrir, d'une manière surprenante, qu'on pouvait obtenir des compositions lavantes, à base de silicones insolubles dans l'eau, en utilisant comme agent dispersant et détergent, un composé acide éther carboxylique polyoxyalkyléné ou l'un de ses sels

L'invention a donc pour objet de nouvelles compositions lavantes, à usage cosmétique ou dermatologique, à base de silicones insolubles dans l'eau et d'au moins un agent tensio-actif du type acide éther carboxylique polyoxyalkyléné ou ses sels cosmétiquement acceptables. Ces compositions sont particulièrement stables.

L'invention a également pour objet des procédés de lavage et/ou de conditionnement des cheveux ou de la peau mettant en oeuvre ces compositions.

D'autres objets apparaîtront à la lecture de la description et des exemples qui suivent.

La présente invention concerne une composition lavante pour la peau ou les cheveux, caractérisée par le fait qu'elle comporte dans un milieu aqueux :

a) une silicone insoluble dans ledit milieu et non réactive avec celui-ci;

b) au moins 7% en poids d'un tensio-actif acide éther carboxylique polyoxyalkylèné, répondant à la formule suivante :

$$R\text{-}(OC_3H_6)_p\text{-}(OC_2H_4)_n\text{-}OCH_2COOA \qquad (I)$$

dans laquelle :

R désigne un radical ou un mélange de radicaux alkyle ou alcényle en $C_8$-$C_{22}$, linéaire ou ramifié, un alkyl($C_8$-$C_9$)phényle, R'CONH-$CH_2$-$CH_2$- avec R' désignant un radical alkyle ou alcényle, linéaire ou ramifié, en $C_{11}$-$C_{21}$;

n est un nombre entier ou décimal compris entre 2 et 24,

p est un nombre entier ou décimal compris entre 0 et 6,

A désigne un atome d'hydrogène ou bien Na, K, Li, 1/2 Mg ou un reste monoéthanolamine, ammonium ou triéthanolamine,

le rapport en poids : composé de formule (I)/silicone insoluble étant supérieur à 1 et de préférence supérieur ou égal à 1,5.

Les silicones, utilisées conformément à la présente invention, sont des polyorganosiloxanes ne portant pas de groupement ammonium quaternaire, insolubles dans les milieux aqueux, pouvant se présenter sous forme d'huiles, de cires, de gommes ou de résines.

Les organopolysiloxanes sont définis plus en détail dans l'ouvrage de Walter NOLL "Chemistry and Technology of Silicones" (1968) Academic-Press.

Ces silicones ne doivent pas être réactives avec lesdits milieux.

Les polyorganosiloxanes plus particulièrement utilisés, conformément à l'invention, sont choisis parmi les silicones volatiles possédant un point d'ébullition compris entre 60°C et 260°C, ou bien des silicones non volatiles choisies en particulier parmi les polyalkylsiloxanes, les polyarylsiloxanes, les polyalkylarylsiloxanes, des gommes et résines de silicones, des polysiloxanes organomodifiés ainsi que leurs mélanges.

Les silicones volatiles sont plus particulièrement choisies parmi :

(i) les silicones cycliques comportant de 3 à 7 atomes de silicium et de préférence 4 à 5. Il s'agit, par exemple, de l'octaméthylcyclotétrasiloxane vendu sous le nom de VOLATILE SILICONE 7207 par UNION CARBIDE ou SILBIONE 70045 V 2 par RHONE POULENC, le décaméthylcyclopentasiloxane vendu sous le nom de VOLATILE SILICONE 7158 par UNION CARBIDE, SILBIONE 70045 V 5 par RHONE POULENC, ainsi que leurs mélanges.

On cite également les cyclocopolymères du type diméthylsiloxane/méthylalkylsiloxane, tel que la SILICONE VOLATILE FZ 3109 vendue par la Société UNION CARBIDE, de structure chimique :

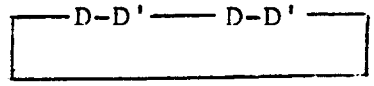

avec D : 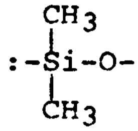 D' : 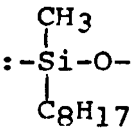

On peut également citer les mélanges silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy)bisnéopentane;

(ii) les silicones volatiles linéaires ayant 2 à 9 atomes de silicium et possédant une viscosité inférieure ou égale à $5.10^{-6}$ $m^2/s$ à 25°C. Il s'agit, par exemple, de l'hexaméthyldisiloxane vendu sous la dénomination SILBIONE 70 041 V 0,65 par la Société RHONE POULENC, du décaméthyltétrasiloxane vendu sous la dénomination SH 200 par la Société TORAY SILICONE ou les polyméthylphénylsiloxanes volatils tels que le produit SILICONOL AS vendu par la Société WACKER. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Les silicones non volatiles sont choisies notamment parmi les polyalkylsiloxanes. On peut citer principalement les polydiméthylsiloxanes linéaires à groupements terminaux triméthylsilyle de viscosité $5.10^{-6}$ à 2,5 $m^2/s$ à 25°C et de préférence $10^{-5}$ à 1 $m^2/s$, comme par exemple et à titre non limitatif :

. les huiles SILBIONE des séries 47 et 70 047 commercialisées par RHONE POULENC telles que l'huile 47 V 500.000,
. les huiles de la série 200 de la Société DOW CORNING,
. les huiles VISCASIL de la GENERAL ELECTRIC et certaines huiles des séries SF de la GENERAL ELECTRIC (SF 96, SF 18).

On cite également les polydiméthylsiloxanes linéaires à groupements terminaux diméthylsilanol, tels que les huiles de la série 48 de RHONE POULENC.

Dans cette classe de polyalkylsiloxanes, on peut également mentionner les cires de polyalkylsiloxanes vendues par la Société GOLDSCHMIDT sous les dénominations ABIL WAX 9800 et ABIL WAX 9801, qui sont des polyalkyl($C_1$-$C_{20}$)siloxanes.

Parmi les polyalkylarylsiloxanes, on peut citer les poly(diméthyl méthyl phényl siloxanes), les polyméthylphénylsiloxanes, les polydiméthyldiphénylsiloxanes linéaires et/ou ramifiés, de viscosité $10^{-5}$ à $5.10^{-2}$ $m^2/s$ à 25°C, tels que, par exemple :

. l'huile RHODORSIL 763 de RHONE POULENC,
. les huiles RHODORSIL de la série 70 633 de RHONE POULENC telles que l'huile RHODORSIL 70 633 V 30,
. les huiles SILBIONE de la série 70 641 de RHONE POULENC telles que l'huile SILBIONE 70 641 V 30 et 70 641 V 200,
. le produit DC 556 COSMETIC GRAD FLUID de DOW CORNING,
. les silicones des séries PK de BAYER, telles que la PK20,
. les silicones des séries PN, PH de BAYER, comme les PN 1000 et PH 1000,
. certaines huiles des séries SF de GENERAL ELECTRIC, telles que les SF 1250, SF 1265, SF 1154, SF 1023.

Les gommes de silicones, conformes à la présente invention, sont des polydiorganosiloxanes de fortes masses moléculaires, comprises entre 200.000 et 1.000.000, utilisées seules ou en mélange dans un solvant choisi parmi les silicones volatiles telles que définies ci-dessus, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On cite, par exemple, les gommes suivantes :

- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)].

On peut citer, par exemple, à titre non limitatif, les mélanges suivants :

. les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA), tel que le produit Q2 1401 vendu par la Société DOW CORNING,

. les mélanges formés à partir d'une gomme polydiméthylsiloxane avec une silicone cyclique, tel que le produit SF 1214 SILICONE FLUID de GENERAL ELECTRIC, qui est une gomme SE 30 correspondant à une Diméthicone vendue par la Société GENERAL ELECTRIC, de poids moléculaire 500.000 solubilisée dans la SF 1202 SILICONE FLUID (correspondant au décaméthylcyclopentasiloxane),

. les mélanges de deux PDMS de viscosités différentes, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus, d'une viscosité de 20 $m^2/s$ et d'une huile SF 96 SILICONE FLUID d'une viscosité de $5.10^{-6}$ $m^2/s$ (15% de gomme SE 30 et 85% d'huile SF 96). Le produit CF 1241 est le mélange d'une gomme SE 30 (33%) et d'une huile PDMS (67%) de viscosité de $10^{-3}$ $m^2/s$.

Les résines d'organopolysiloxanes, conformes à la présente invention, sont des systèmes siloxaniques réticulés renfermant les unités $R'_2SiO_{2/2}$, $R'SiO_{3/2}$ et $SiO_{4/2}$, dans lesquelles R' représente un groupement hydrocarboné possédant 1 à 6 atomes de carbone ou un groupement phényle.

Parmi ces produits, ceux particulièrement préférés sont ceux dans lesquels R' désigne un radical alkyle inférieur ou phényle.

Parmi ces résines, on peut citer le produit vendu sous la dénomination DOW CORNING 593 ou ceux vendus sous les dénominations SILICONES FLUID SS 4230 et SS 4267 de la GENERAL ELECTRIC qui sont des diméthyl/triméthylpolysiloxanes.

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite les silicones comportant :

1- des groupements polyéthylèneoxy et/ou polypropylèneoxy, comportant éventuellement des groupes alkyles, tels que :

. le produit dénommé diméthicone copolyol vendu par la Société DOW CORNING sous la dénomination DC 1248, et l'alkyl($C_{12}$)méthicone copolyol vendue par la Société DOW CORNING sous la dénomination Q2 5200,

. les huiles SILWET L 722, L 7500, L 77, L 711 de la Société UNION CARBIDE,

. le mélange de diméthicone copolyol et de cyclométhicone tel que le produit vendu sous la dénomination Q2-3225C par la Société DOW CORNING,

2- des groupements aminés, substitués ou non, comme dans la GP4 SILICONE FLUID de GENESEE, la GP 7100 de GENESEE ou les produits vendus sous les dénominations Q2 8220 et Q2 8200, par la Société DOW CORNING. Les groupements aminés substitués sont en particulier des groupements aminoalkyle($C_1$-$C_4$),

3- des groupements thiols comme dans les produits GP 72 A et GP 71 de GENESEE ou dans le produit SLM 50253/5 de la Société WACKER,

4- des groupements carboxylates comme dans le cas des produits décrits dans le brevet européen EP-A-186 507 de CHISSO CORPORATION,

5- des groupements hydroxylés comme les polyorganosiloxanes à fonction hydroxyalkyle, décrits dans la demande de brevet français n° FR-85 163 34, répondant à la formule suivante :

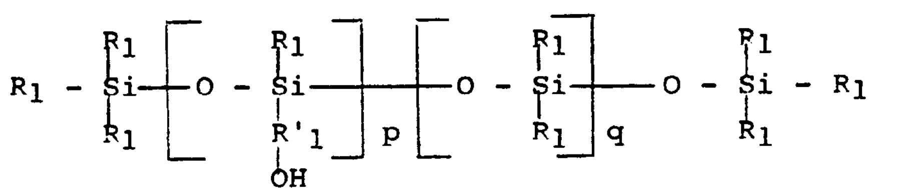 (II)

dans laquelle :

- les radicaux $R_1$, identiques ou différents, sont choisis parmi les radicaux méthyle et phényle, au moins 60% en moles des radicaux $R_1$ étant méthyle;
- le radical $R'_1$ est un chaînon alkylène divalent hydrocarboné en $C_2$-$C_{18}$;
- p est compris entre 1 et 30 inclus;
- q est compris entre 1 et 150 inclus.

On cite, par exemple, le produit 71 615 V 300 vendu par la société RHONE POULENC.

6- des groupements alcoxylés comme le produit vendu sous la dénomination SILICONE COPOLYMER F-755 par la Société SWS SILICONES et ABIL WAX 2428, 2434 et 2440 par la Société GOLDSCHMIDT,

7- des groupements acyloxyalkyle, comme par exemple les polyorganosiloxanes décrits dans la demande de brevet français n° FR-2.641.185, répondant à la formule suivante :

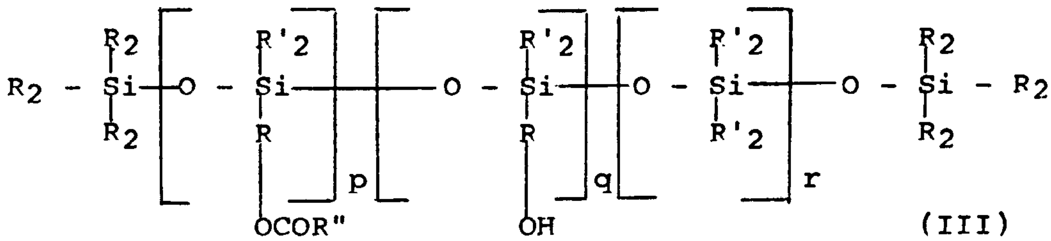

(III)

dans laquelle :

- $R_2$ désigne méthyle, phényle, -OCOR", hydroxyle, un seul des $R_2$ par atome de silicium peut être OH;
- $R'_2$ désigne méthyle, phényle, 60% molaire au moins de l'ensemble des radicaux $R_2$ et $R'_2$ est méthyle;
- R" désigne alkyle ou alcényle en $C_8$-$C_{20}$;
- R désigne un alkylène hydrocarboné divalent, linéaire ou ramifié, en $C_2$-$C_{18}$;
- r est compris entre 1 et 120 inclus;
- p est compris entre 1 et 30;
- q vaut 0 ou est inférieur à 0,5 p, p+q étant compris entre 1 et 30; les polyorganosiloxanes de formule (III) peuvent contenir des groupements

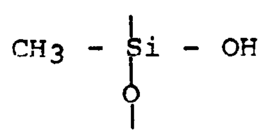

dans des proportions ne dépassant pas 15% de la somme p+q+r.

Les composés de formule (III) peuvent être préparés par estérification de polyorganosiloxanes à fonction hydroxyalkyle de formule (II) ci-dessus.

L'estérification s'effectue de façon connue, avec un acide R"COOH ou l'anhydride d'acide correspondant à une température comprise entre 100 et 250°C en présence éventuellement d'un catalyseur comme le chlorure d'aluminium ou le chlorure de zinc et d'un acide fort comme l'acide chlorhydrique ou l'acide sulfurique.

On peut également effectuer une transestérification par chauffage à 100-150°C d'un ester méthylique de formule R"$COOCH_3$ et d'un diorganopolysiloxane de formule (II), en présence d'un catalyseur acide comme l'acide paratoluènesulfonique ou une terre acide de type Montmorillonite (KATALYSATOR KSF/O, vendu par SUD-CHEMIE - A.G. MUNCHEN).

8- des groupements 2-hydroxyalkylthiosulfate tels que dans les produits vendus par la Société GOLDSCHMIDT sous les dénominations ABIL S 201 et ABIL S 255.

9- des groupements anioniques de type carboxylique tels que les groupements alkylcarboxyliques comme ceux présents dans le produit X-22-3701E de la Société PHIN-ETSU ou dans le produit Silicone Fluid FZ 3703 de la Société UNION CARBIDE; 2-hydroxyalkylsulfonate.

Les acides éthers carboxyliques polyoxyalkylénés utilisés, conformément à l'invention, sont choisis de préférence parmi les composés de formule (I) dans laquelle R désigne un radical ou un mélange de radicaux alkyl($C_{12}$-$C_{14}$), oléyle, cétyle, stéaryle; un radical nonylphényle ou octylphényle, A désigne un atome d'hydrogène ou de sodium et p=0. n varie de 2 à 20 et de préférence de 2 à 10.

On utilise de préférence les produits commerciaux vendus par la Société CHEM Y sous les dénominations :

AKYPO NP 70 (R=nonylphényle, n=7,p=0,A=H)
AKYPO NP 40 (R=nonylphényle, n=4,p=0,A=H)
AKYPO OP 40 (R=octylphényle, n=4,p=0,A=H)
AKYPO OP 80 (R=octylphényle, n=8,p=0,A=H)
AKYPO OP 190 (R=octylphényle, n=19,p=0,A=H)
AKYPO RLM 25 (R=alkyle($C_{12}$-$C_{14}$),n=2,5,p=0,A=H)

AKYPO RLM 38 (R=alkyle($C_{12}$-$C_{14}$),n=3,8,p=0,A=H)
AKYPO RLMQ 38 NV (R=alkyle($C_{12}$-$C_{14}$),n=4,p=0, A=Na)
AKYPO RLM 45 (R=alkyle($C_{12}$-$C_{14}$),n=4,5,p=0,A=H)
AKYPO RLM 45 NV (R=alkyle($C_{12}$-$C_{14}$),n=5,p=0, A=Na)
AKYPO RLM 100 (R=alkyle($C_{12}$-$C_{14}$),n=10,p=0,A=H)
AKYPO RLM 100 NV (R=alkyle($C_{12}$-$C_{14}$),n=10,p=0, A=Na)
AKYPO RLM 130 (R=alkyle($C_{12}$-$C_{14}$),n=13,p=0,A=H)
AKYPO RLM 160 NV (R=alkyle($C_{12}$-$C_{14}$),n=16,p=0, A=Na)
AKYPO RO 20 (R=oléyle, n=2,p=0,A=H)
AKYPO RO 90 (R=oléyle,n=9,p=0,A=H)
AKYPO RCS 60 (R=cétyle/stéaryle,n=6,p=0,A=H)
AKYPO RS 60 (R=stéaryle,n=6,p=0,A=H)
AKYPO RS 100 (R=stéaryle,n=10,p=0,A=H)
AKYPO RO 50 (R=oléyle,n=5,p=0,A=H)

ou par la Société SANDOZ sous les dénominations :

SANDOPAN ACA-48 (R=cétyle/stéaryle,n=24,p=0, A=H)
SANDOPAN DTC Acid (R=alkyle($C_{13}$),n=6,p=0,A=H)
SANDOPAN DTC (R=alkyle($C_{13}$),n=6,p=0,A=Na)
SANDOPAN LS-24 (R=alkyle($C_{12}$-$C_{14}$),n=12,p=0, A=Na)
SANDOPAN JA-36 (R=alkyle($C_{13}$),n=18,p=0,A=H),

et plus particulièrement les produits vendus sous les dénominations suivantes :

AKYPO NP 70
AKYPO NP 40
AKYPO OP 40
AKYPO OP 80
AKYPO RLM 25
AKYPO RLM 45
AKYPO RLM 100
AKYPO RO 20
AKYPO RO 50
AKYPO RLM 38

Dans les compositions conformes à l'invention, les composés de formule (I) sont présents dans des proportions comprises entre 7% et 50% et de préférence entre 8% et 30% par rapport au poids total des compositions.

Les silicones sont présentes dans des proportions comprises entre 0,2 et 30% et de préférence entre 0,2 et 10% par rapport au poids total des compositions.

Les compositions cosmétiques, selon l'invention, peuvent être utilisées en particulier comme shampooings, crème lavante, sous forme de gels douche ou de bains moussants pour le corps.

Les compositions cosmétiques ou dermatologiques, conformes à la présente invention, peuvent contenir, en outre, d'autres agents tensio-actifs, différents de ceux de formule (I), choisis parmi les agents tensio-actifs anioniques, amphotères, zwittérioniques, non ioniques ou leurs mélanges.

Parmi les tensio-actifs anioniques, on peut citer plus particulièrement : les sels alcalins, les sels d'ammonium, les sels d'amines ou les sels d'aminoalcools des composés suivants :

- les alkylsulfates, alkyléthersulfates, alkylamidesulfates et éthersulfates, alcanolamidesulfates, alkylarylpolyéthersulfates, monoglycéridesulfates,
- les alkylsulfonates, alkylamidesulfonates, alkylarylsulfonates, oléfinesulfonates, paraffinesulfonates,
- les alkylsulfosuccinates, alkyléthersulfosuccinates, alkylamidesulfosuccinates,
- les alkylsulfosuccinamates,
- les alkylsulfoacétates,
- les acylsarcosinates, acylpolypeptidates, acylamidopolypeptidates, acyliséthionates, N-acyltaurates;

le radical alkyle ou acyle de ces composés étant constitué par une chaîne carbonée comportant 8 à 18 atomes de carbone.

Parmi les agents tensio-actifs anioniques, on peut également citer :

- les sels d'acides gras tels que les acides oléique, ricinoléique, palmitique, stéarique, les acides d'huile de coprah ou d'huile de coprah hydrogénée.
- des acyl lactylates, dont le radical acyle comporte 8 à 20 atomes de carbone.

Parmi les agents tensio-actifs non ioniques, on peut citer en particulier : les alcools, les alkylphénols et acides gras polyoxyéthylénés, polypropoxylés ou polyglycérolés à chaîne grasse comportant 8 à 18 atomes

de carbone, le nombre de groupements d'oxyde d'éthylène ou d'oxyde de propylène étant compris entre 2 et 50 et le nombre de groupements glycérol étant compris entre 2 et 30.

On peut également citer les copolymères d'oxydes d'éthylène et de propylène, des condensats d'oxydes d'éthylène et de propylène sur des alcools gras, des amides gras polyéthoxylés (de préférence de 2 à 30 moles d'oxyde d'éthylène), des éthanolamides, des esters d'acides gras de glycol, des esters d'acides gras du sorbitan oxyéthylénés (de préférence de 2 à 30 moles d'oxyde d'éthylène) ou non, des esters d'acides gras du saccharose, des esters d'acides gras de polyéthylène glycol, des esters d'acides gras de dérivés du glucose, des amines grasses polyéthoxylées (de préférence de 2 à 30 moles d'oxyde d'éthylène), des oxydes d'amines, tels que les oxydes d'alkylamine ou de N-acylamidopropylmorpholine.

Les alcools gras oxyéthylénés ou polyglycérolés préférés sont l'alcool oléique oxyéthyléné à 10 moles d'oxyde d'éthylène, l'alcool laurique oxyéthyléné à 12 moles d'oxyde d'éthylène, le nonylphénol oxyéthyléné à 9 moles d'oxyde d'éthylène, l'alcool oléique polyglycérolé à 4 moles de glycérol; l'ester d'acide gras du sorbitan polyoxyéthyléné préféré est le monolaurate de sorbitan polyoxyéthyléné à 20 moles d'oxyde d'éthylène.

D'autres composés entrant dans cette classe sont des composés poly(hydroxypropyl)éthers répondant aux formules (IV) à (VI) ci-après et/ou préparés selon les procédés décrits ci-après :

$$(1)\quad R_4O-(CH_2-\underset{\displaystyle CH_2OH}{\underset{|}{CH}}-O)_m-H \qquad (IV)$$

dans laquelle $R_4$ désigne un radical ou un mélange de radicaux alkyles contenant 10 à 14 atomes de carbone et m est un nombre entier ou décimal de 2 à 10 et de préférence de 3 à 6. Ces composés de formule (IV) peuvent être préparés selon le procédé décrit dans le brevet FR-A-1 477 048;

$$(2)\quad R_5-CONH-CH_2-CH_2-O-CH_2-CH_2-O-(CH_2-CHOH-CH_2-O)_nH \qquad (V)$$

dans laquelle $R_5$ désigne un radical ou un mélange de radicaux alkyles et/ou alcényles ayant de 11 à 17 atomes de carbone et n désigne un nombre entier ou décimal de 1 à 5, et de préférence 1,5 à 4. Ces composés de formule (V) peuvent être préparés selon le procédé décrit dans le brevet FR-A-2 328 763;

$$(3)\quad R_6-CHOH-CH_2-O-(CH_2-CHOH-CH_2-O)_p-H \qquad (VI)$$

dans laquelle $R_6$ désigne un radical aliphatique, cycloaliphatique, arylaliphatique, ayant de préférence 7 à 21 atomes de carbone, et leurs mélanges, les chaînes aliphatiques désignant en particulier des chaînes alkyles pouvant comporter 1 à 6 groupements éther, thioéther et/ou hydroxyméthylène et p est compris entre 1 et 10 inclus.

Ces composés sont préparés par condensation en catalyse alcaline, de 2 à 10 et de préférence de 2,5 à 6 moles de glycidol sur un alpha-diol ou un mélange d'alpha-diols en $C_{10}$-$C_{14}$, à la température de 120-180°C et de préférence de 140 à 160°C, le glycidol étant ajouté lentement selon le procédé de préparation décrit dans le brevet FR-A-2 091 516;

(4) Les composés préparés par condensation, en catalyse acide, de 2 à 10 et de préférence de 2,5 à 6 moles de glycidol par mole d'alcool ou d'alpha-diol contenant 10 à 14 atomes de carbone à une température de 50 à 120°C, le glycidol étant ajouté lentement à l'alcool ou à l'alpha-diol. Le procédé de préparation de ces composés est décrit plus particulièrement dans le brevet FR-A-2 169 787;

(5) Les composés de poly(hydroxypropyl)éthers préparés par polyaddition de monochlorhydrine du glycérol sur un composé organique (poly)hydroxylé en présence d'une base forte, avec élimination au fur et à mesure de l'eau par distillation, décrits en particulier dans le brevet français FR-A-2 574 786.

Parmi les tensio-actifs non-ioniques de la famille des poly(hydroxypropyl)éthers décrits dans les paragraphes (1), (2), (3), (4) et (5) ci-dessus, les composés préférés sont représentés par les formules :

$$(\alpha)\quad \cdot\ C_{12}H_{25}O-(CH_2-\underset{\displaystyle CH_2OH}{\underset{|}{CH}}-O)_{4,2}-H \qquad (VII)$$

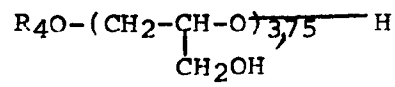 (VIII)

où $R_4$ désigne un mélange de radicaux alkyles $C_{10}H_{21}$ et $C_{12}$-$H_{25}$;

(β) . les composés préparés par condensation en catalyse alcaline, de 3,5 moles de glycidol sur un alpha-diol ayant 12 atomes de carbone, selon le procédé décrit dans le brevet FR-A-2 091 516;

(γ) . les composés répondant à la formule :

$$R_6-CONH-CH_2-CH_2-O-CH_2-CH_2-O-(CH_2-CHOH-CH_2-O)_{3,5}-H \quad (IX)$$

où $R_6$ désigne un mélange de radicaux comprenant les radicaux alkyles et alcényles suivants : $C_{11}H_{23}$, $C_{13}H_{27}$, les radicaux dérivés des acides gras du coprah et le radical dérivé de l'acide oléique;

(δ) . les composés préparés par condensation de 3,5 moles de glycidol sur un mélange d'alpha-diols en $C_{11}$-$C_{14}$, décrits dans le brevet FR-A-2 091 516, le tensio-actif non-ionique poly(hydroxypropyl)éther obtenu par condensation de la monochlorhydrine du glycérol (2,5 moles) en présence de soude sur le dodécanediol-1,2, sont plus particulièrement préférés.

Parmi les agents tensio-actifs amphotères et zwittérioniques qui peuvent être utilisés, on peut citer par exemple :

1. les dérivés d'amines secondaires ou tertiaires aliphatiques, dans lesquels le radical aliphatique est une chaîne linéaire ou ramifiée comportant de 6 à 18 atomes de carbone et qui contient au moins un groupe anionique hydrosoluble carboxylique, sulfonate, sulfate, phosphate ou phosphonate.

Parmi ces composés, on peut citer plus particulièrement les produits vendus sous la dénomination MIRANOL, décrits dans les brevets US-A-2 528 378 et 2 781 354 et classés dans le dictionnaire CTFA, 3ème édition, 1982, sous la dénomination des Amphocarboxyglycinates et Amphocarboxypropionates.

Ces produits présentent les structures suivantes :

AMPHOCARBOXYGLYCINATE

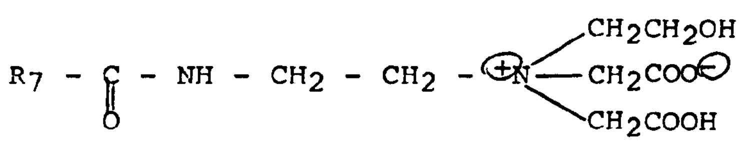

dans laquelle $R_7$ désigne un radical alkyle dérivé du coprah, un radical heptyle, nonyle ou undécyle;

AMPHOCARBOXYPROPIONATE

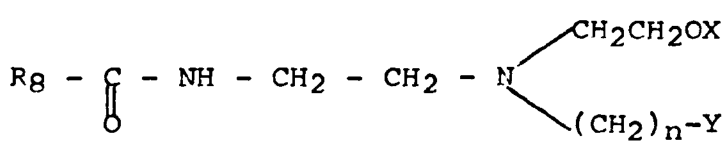

dans laquelle :

n vaut 1 ou 2;

X désigne le groupement $-CH_2CH_2COOH$ ou hydrogène;

Y désigne -COOH ou le radical

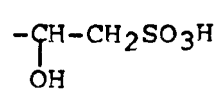

$R_6$ désigne un radical alkyle dérivé du coprah, un radical alkyle en $C_7$, $C_9$, $C_{11}$, $C_{13}$, un radical alkyle en $C_{17}$ et sa forme iso, un radical en $C_{17}$ insaturé, un radical alkyle dérivé de l'huile de lin;

2. les alkylbétaïnes, les sulfobétaïnes, les amidobétaïnes ou les amidosulfobétaïnes.

Les alkylbétaïnes sont choisies de préférence parmi les alkyl($C_{10}$-$C_{20}$)bétaïnes.

Conformément à la présente invention, on utilise de préférence en association avec les composés de formule (I), des mélanges de tensio-actifs et en particulier des mélanges constitués de tensio-actifs anioniques ou des mélanges constitués des tensio-actifs anioniques associés à des agents tensio-actifs amphotères, zwittérioniques ou non-ioniques.

On utilise de préférence un ou plusieurs agents tensio-actifs anioniques choisis parmi les alkyl($C_{12}$-$C_{14}$)sulfates de sodium, de triéthanolamine ou d'ammonium, les alkyl($C_{12}$-$C_{14}$)éthersulfates de sodium oxyéthylénés à 2,2 moles d'oxyde d'éthylène, le cocoyliséthionate de sodium, l'$\alpha$-oléfine($C_{14}$-$C_{16}$) sulfonate de sodium, le lauroylsarcosinate de sodium et leurs mélanges avec

. soit un tensio-actif amphotère tel qu'un amphocarboxyglycinate défini par la formule ci-dessus, dans laquelle $R_7$ désigne un radical alkyle dérivé du coprah, dénommé Cocoamphocarboxyglycinate vendu par la Société MIRANOL sous la dénomination commerciale MIRANOL C2M CONC;

. soit un tensio-actif zwittérionique tel que la laurylbétaïne vendue par la Société HENKEL sous la dénomination commerciale DEHYTON AB 30;

. soit un tensio-actif non-ionique de formules (IV), (V), (VI) ci-dessus ou un oxyde d'alkylamine.

Lorsque les tensio-actifs amphotères ou zwittérioniques sont utilisés en mélange avec les tensio-actifs anioniques, ils représentent jusqu'à 50%, et de préférence de 5 à 30% en poids du poids total de la quantité d'agents tensio-actifs présents dans la composition.

Lorsque les tensio-actifs non-ioniques sont utilisés en mélange avec des tensio-actifs anioniques, ils représentent jusqu'à 80%, et de préférence de 5 à 50% du poids total de la quantité d'agents tensio-actifs présents dans la composition.

Les compositions cosmétiques ou dermatologiques selon la présente invention, peuvent se présenter sous la forme de produits opaques ou limpides.

Pour obtenir des compositions opaques, on préfère utiliser des silicones choisies parmi :

. les huiles de silicone de forte viscosité comprise entre 0,2 et 2,5 $m^2/s$ à 25°C, telles que les huiles 47 V 500.000 de la Société RHONE POULENC.

. les mélanges d'organopolysiloxanes et de silicones cycliques, tels que le produit Q2 1401 vendu par la Société DOW CORNING.

. les mélanges de deux PDMS de viscosités différentes, tels que le produit vendu par la Société GENERAL ELECTRIC sous la dénomination CF 1241.

. les huiles organomodifiées telles que les silicones à groupement $\gamma$-hydroxypropyle, en particulier l'huile 71 615 V 300 vendue par la Société RHONE POULENC, ou à fonction acyloxyalkyle en particulier stéaroyloxypropyle décrite ci-dessus.

Pour obtenir des produits limpides, on préfère utiliser selon l'invention, des silicones choisies parmi :

. les silicones volatiles cycliques ayant de 3 à 7 atomes de silicium, telles que l'octaméthylcyclotétrasiloxane vendu sous la dénomination HUILE SILBIONE 70045 V 2 de la Société RHONE POULENC ou le décaméthylcyclopentasiloxane vendu sous la dénomination VOLATILE SILICONE 7158 de la Société UNION CARBIDE ou leurs mélanges avec des composés organosiliciés.

. les huiles PPMS de viscosité inférieure à $5.10^{-5}$ $m^2/s$ telles que les huiles 70 633 V 30 de la Société RHONE POULENC, DC 556 de la Société DOW CORNING.

. les huiles PDMS linéaires ayant de 2 à 9 atomes de silicium et une viscosité inférieure à $2.10^{-6}$ $m^2/s$ telles que l'huile SH 200 de la Société TORAY SILICONE.

Parmi les silicones ci-dessus, on préfère plus particulièrement les silicones volatiles cycliques et les PPMS de viscosité inférieure à $5.10^{-5}$ $m^2/s$.

Pour les compositions opaques conformes à la présente invention, on utilise plus particulièrement des composés de formule (I), pour lesquels :

. R = octylphényle
n = 4, p=0
A = H

Ce composé est vendu sous la dénomination AKYPO OP 40 par la Société CHEM Y à 90% de matière active (MA).

. R = nonylphényle
n = 4, p=0
A = H

Ce composé est vendu sous la dénomination AKYPO NP 40 par la Société CHEM Y à 90% de MA.

. R = oléyle
n = 2 ou 5, p=0

A = H

Ces composés sont vendus sous les dénominations AKYPO RO 20 et AKYPO RO 50 par la Société CHEM Y à 90% de MA.

. R = alkyle en $C_{12}$-$C_{14}$

n = 2,5

A = H

Ce composé est vendu sous la dénomination AKYPO RLM 25 par la Société CHEM Y à 90% de MA.

Pour les compositions limpides conformes à la présente invention, on utilise plus particulièrement des composés de formule (I), pour lesquels :

. R = nonylphényle

n = 7, p=0

A = H

Ce composé est vendu sous la dénomination AKYPO NP 70 par la Société CHEM Y, à 90% de MA, ou un mélange de ces composés, pour lesquels :

. R = octylphényle

n = 4 ou 8, p=0

A = H

Ces composés sont vendus sous les dénominations AKYPO OP 40 et AKYPO OP 80 par la Société CHEM Y, et

. R = lauryle

n = 10, p=0

A = H

Ce composé étant vendu sous la dénomination AKYPO RLM 100 par la Société CHEM Y à 90% de MA.

La quantité totale en tensio-actifs dans ces compositions de lavage est comprise généralement entre 7 et 50% en poids par rapport au poids total de la composition et en particulier entre 8 et 30% en poids.

La proportion en tensio-actif additionnel ne dépasse pas 40% et est de préférence comprise entre 0 et 20%.

Les compositions cosmétiques ou dermatologiques, selon l'invention, présentent un pH généralement compris entre 2 et 9.

Les compositions selon l'invention peuvent se présenter sous forme de liquides plus ou moins épaissis, de gels ou de mousses aérosols.

Elles peuvent contenir également des agents régulateurs de viscosité, tels que des électrolytes, des hydrotropes ou d'autres épaississants dont on peut citer par exemple : le chlorure de sodium, le xylènesulfonate de sodium, les dérivés de la cellulose, tels que par exemple la carboxyméthylcellulose, l'hydroxypropylcellulose, la gomme de guar, des gommes de guar hydroxypropylées et les scléroglucanes.

Ces agents régulateurs de viscosité sont utilisés dans des proportions allant jusqu'à 15% en poids par rapport au poids total de la composition et de préférence inférieure à 6%.

Les compositions conformes à l'invention peuvent éventuellement contenir en outre d'autres agents ayant pour effet d'améliorer les propriétés cosmétiques des cheveux et/ou de la peau, pourvu qu'ils n'altèrent pas la stabilité des compositions, tels que des tensio-actifs cationiques, des polymères anioniques ou cationiques ou des protéines quaternisées ou non et des silicones hydrosolubles.

Les polymères cationiques ou anioniques, les tensio-actifs cationiques et les protéines quaternisées ou non, sont utilisés dans les compositions cosmétiques ou dermatologiques, selon l'invention, dans des proportions comprises entre 0,05 et 6% et de préférence entre 0,1 et 3% par rapport au poids total de la composition.

Les silicones hydrosolubles peuvent être utilisées dans toutes les compositions selon l'invention, dans des proportions allant jusqu'à 10% et de préférence entre 0,5 et 6% par rapport au poids total de la composition.

Les compositions selon l'invention peuvent également contenir différents adjuvants habituellement utilisés en cosmétique, tels que des parfums, des conservateurs, des séquestrants, des stabilisateurs de mousse, des agents propulseurs, des colorants, des agents acidifiants ou alcalinisants ou d'autres adjuvants selon l'usage envisagé.

Les compositions dermatologiques contiennent en outre une substance active pour le traitement des affections dermatologiques.

Les procédés de lavage et de conditionnement des cheveux ou de la peau consistent à appliquer sur ceux-ci une composition telle que définie ci-dessus et choisie selon le traitement envisagé, cette application étant suivie d'un rinçage.

EXEMPLES 1 à 5

On prépare des shampooings de compositions suivantes :

TABLEAU I

| en g MA | 1 | 2 | 3 | 4 | 5 |
|---|---|---|---|---|---|
| AKYPO NP 70 à 90% de MA | 18 | | 13,5 | 9 | 10,8 |
| AKYPO OP 40 à 90% de MA | | | | 4,5 | |
| AKYPO RO 20 à 90% de MA | | 3,6 | | | |
| AKYPO RO 90 à 90% de MA | | 3,6 | | | |
| Huile 47 V 500 000 RP | | | | 1 | |
| Silbione 70 047 V 300 RP | 10 | 2 | | | |
| Silbione 70 633 V 30 RHONE POULENC | | | | | 2 |
| Mélange d'un diméthiconol et d'une cyclométhicone vendu sous la dénomination Q2 - 1401 par DOW CORNING | | | 1 | | |
| Eau qsp | 100 | 100 | 100 | 100 | 100 |
| Triéthanolamine qs pH | 4 | | | | |
| pH spontané | | 2,7 | 2,2 | 2,2 | 2,3 |

Exemples 6 à 12 :

On prépare des shampooings limpides dont les compositions sont indiquées dans le tableau 2 :

Exemples 13 à 19 :

On prépare des shampooings opaques dont les compositions sont indiquées dans le tableau 3 :

TABLEAU 2 : Shampooings limpides

| en g MA | 6 | 7 | 8 | 9 | 10 | 11 | 12 |
|---|---|---|---|---|---|---|---|
| AKYPO NP 70 | 9 | 9 | 9 | | 7,2 | 9 | 9 |
| AKYPO OP 40 | | | | 6,3 | | | |
| AKYPO OP 80 | | | | 9 | | | |
| AKYPO RLM 100 | | | | 2,7 | | | |
| Décaméthylcyclopentasiloxane vendu par la Société UNION CARBIDE sous la dénomination Volatile Silicone 7158 | 2,5 | | | | | | |
| Décaméthyltétrasiloxane vendu par la Société TORAY SILICONE sous la dénomination SH 200 | | 2,5 | | | | | |
| Huile 70 633 V 30 (RHONE POULENC) | | | | 5 | 2 | | |
| Mélange de $D_4$et de composé silicié (1) | | | | | | | 2,5 |
| Mélange de $D_4$et de composé silicié (2) | | | 2,5 | | | 2,5 | |
| Diméthicone copolyol vendu à 35% de MA par la Société GOLDSCHMIDT sous la dénomination Huile CL 183/25 | | | | | 5 | | |
| Alkyl($C_{12}$-$C_{14}$)éther sulfate de sodium oxyéthyléné à 2,2 moles d'oxyde d'éthylène vendu à 25% de MA | 10 | 10 | | | | | |
| Alkyl($C_{12}$-$C_{14}$/70-30)sulfate de triéthanolamine en solution aqueuse à 40% de MA | | | 10 | 10 | 10 | 4 | 12 |
| Laurylbétaïne en solution aqueuse à 32% de MA vendue sous la dénomination DEHYTON AB 30 par la Société HENKEL | | | | | | | 3 |

TABLEAU 2 (suite)

| en g MA | 6 | 7 | 8 | 9 | 10 | 11 | 12 |
|---|---|---|---|---|---|---|---|
| Lauroylsarcosinate de sodium vendu en solution aqueuse à 30% de MA par la Société SEPPIC sous la dénomination ORAMIX L30 | | | | | 4 | | |
| Tensio-actif non-ionique poly(hydroxypropyléther) préparé par condensation en catalyse alcaline de 3,5 moles de glycidol sur un alpha-diol ayant 12 atomes de carbone, selon le procédé décrit dans le brevet FR-A-2 091 516 | | | | | | 4 | |
| Polymère d'hydroxyéthyl-cellulose et d'épichlo-rhydrine quaternisé avec la triméthylamine vendu sous la dénomination JR 400 par la Société UNION CARBIDE | | | | | 1 | | |
| Diéthanolamide d'acide de coprah | 2,5 | 2,5 | 2,5 | | | 2,5 | 2,5 |
| Chlorure de sodium | 4 | 4 | 2,5 | | | | |
| Triéthanolamine qs pH | 7,2 | 7,2 | 8 | 7 | 6 | 4 | |
| pH spontané | | | | | | | 3,6 |
| Conservateurs, parfums | qs | qs | qs | qs | qs | qs | qs |
| Eau qsp | 100 | 100 | 100 | 100 | 100 | 100 | 100 |

(1) Mélange d'octaméthylcyclotétrasiloxane ($D_4$) et de tétratriméthylsilylpentaérythritol.

(2) Mélange d'octaméthylcyclotétrasiloxane ($D_4$) et d'oxy-1,1'(hexa-2,2,2',2',3,3'-triméthylsilyloxy)bis-néopentane.

TABLEAU 3 : Shampooings opaques

| en g MA | 13 | 14 | 15 | 16 | 17 | 18 | 19 |
|---|---|---|---|---|---|---|---|
| AKYPO NP 70 | 9 | | | | 4,5 | | |
| AKYPO OP 40 | | | | 9,7 | | 10,8 | 10,8 |
| AKYPO OP 80 | | 36 | | | | | |
| AKYPO NP 40 | 4,5 | | 10,8 | | 4,5 | | |
| Huile 47 V 500 000 (RHONE POULENC) | 1 | | | 1,5 | | | |
| Silicone DC 593 vendue par la Société DOW CORNING | | | | | 0,5 | | |
| Mélange d'un diméthiconol et d'une cyclométhicone vendu sous la dénomination Q2-1401 par la Société DOW CORNING | | | 2 | | | | |
| Mélange de deux PDMS de viscosités différentes vendu sous la dénomination CF 1241 par GENERAL ELECTRIC | | | | | | 3 | |
| Huile 47 V 300 (RHONE POULENC) | | 0,5 | | | | | |
| Huile Silbione 71615 V 300 (RHONE POULENC) | | | | | | | 2 |
| Cocoylisethionate de sodium vendu sous la dénomination FENOPON AC 78 par la Sté GAF | | | 12 | | | | |
| Mélange (84/8 poids) de cocoyl-iséthionate de sodium/isothionate de sodium vendu sous la dénomination ARLATONE SCI par la Société ICI | | 5 | | | | | |
| Laurylsulfate de sodium | | | | | 10 | 15 | |
| Laurylsulfate d'ammonium | | | | 5 | | | |
| Oléfine $C_{14}$-$C_{16}$ sulfonate de sodium vendu en solution aqueuse à 38% de MA | 5,7 | | | 5 | | | |

TABLEAU 3 (suite)

| en g MA | 13 | 14 | 15 | 16 | 17 | 18 | 19 |
|---|---|---|---|---|---|---|---|
| Alkyl($C_{12}$-$C_{14}$/70-30)sulfate de triéthanolamine en solution aqueuse à 40% de MA | | | | | | | 10 |
| Cocoamphocarboxyglycinate vendu sous la dénomination MIRANOL C2M CONC par la Sté MIRANOL en solution aqueuse à 38% de MA | | | | | | 2 | |
| Laurylbétaïne en solution aqueuse à 32% de MA | 3 | | | | 5 | | |
| Tensio-actif non-ionique poly (hydroxypropyléther) préparé par condensation en catalyse alcaline de 3,5 moles de glycidol sur un alphadiol ayant 12 atomes de carbone, selon le procédé décrit dans le brevet FR-A-2 091 516 | | | | | | | 3 |
| (N,N-diméthyl N-alkyl($C_{11}$-$C_{17}$) amidopropyl)amineoxyde vendu en solution aqueuse à 35% de MA par la Société GOLDSCHMIDT sous la dénomination AMINOXID WS 35 | | | | | | | 0,5 |
| Protéine quaternisée vendue sous la dénomination LEXEIN QX 3000 par la Sté INOLEX à 30% de MA | | | | | 0,5 | | |
| Chlorure de stéaryldiméthyl-benzylammonium | | | 1 | | 1 | | |
| Chlorure de sodium | | | 3 | 3 | | 3 | 2 |
| Triéthanolamine qs pH | 5 | | | | | | 4,5 |
| Hydroxyde de sodium qs pH | | | 4 | | | | |
| pH spontané | | 3 | | 3 | 3,1 | 3 | |
| Eau qsp | 100 | 100 | 100 | 100 | 100 | 100 | 100 |

EXEMPLE 20

On prépare un bain moussant de composition suivante :

- AKYPO RLM 45 à 90% de MA 13,5 g MA
- Huile 47 V 500 000 vendue par la Société RHONE POULENC 0,1 g
- n-alkyl($C_{14}$-$C_{17}$)sulfonate de sodium vendu sous la dénomination HOSTAPUR SAS 30 par la Société

HOECHST 18,0 g
- Conservateur, parfum qs
- pH spontané = 5
- Eau qsp 100,0 g

EXEMPLE 21

On prépare un gel douche limpide de composition suivante :
- AKYPO OP 40 à 90% de MA 3,94 g MA
- AKYPO OP 80 à 90% de MA 5,63 g MA
- AKYPO RLM 100 à 90% de MA 1,69 g MA
- Huile SILBIONE 70 633 V 30 (PPMS) vendue par la Société RHONE POULENC 2,5 g
- Alkyl($C_{12}$-$C_{14}$)sulfate de triéthanolamine 16,0 g MA
- Conservateur, parfum qs
- Triéthanolamine qs pH = 7
- Eau qsp 100,0 g

EXEMPLE 22

On prépare une crème lavante de composition suivante :
- AKYPO RO 20 vendu à 90% de MA par la Société CHEM Y 10,8 g MA
- Huile Silbione 70 047 V 300 (PDMS) vendue par la Société RHONE POULENC 10,0 g
- Hydroxyde de sodium qs pH = 5,5
- Eau qsp 100,0 g

EXEMPLE 23

On prépare un shampooing-gel de composition suivante :
- AKYPO RO 50 vendu à 90% de MA par la Société CHEM Y 5,0 g MA
- AKYPO RO 90 vendu à 90% de MA par la Société CHEM Y 20,0 g MA
- Huile Silbione 70 047 V 500.000 (PDMS)vendue par la Société RHONE POULENC 5,0 g
- Chlorure de sodium 5,0 g
- Hydroxyde de sodium qs pH = 3,7
- Eau qsp 100,0 g

EXEMPLE 24

On prépare un bain moussant limpide de composition suivante :
- AKYPO RLM 38 vendu à 90% de MA par la Société CHEM Y 8,0 g MA
- Alkyl($C_9$/$C_{11}$/$C_{13}$/$C_{15}$-3/7/63/27%, à 50% linéaire)éther sulfate de sodium oxyéthyléné à 3 oxyde d'éthylène, vendu en solution aqueuse à 70% de MA par la Société ICI 21,0 g MA
- Huile Q2-8200 (PDMS à fonction amine) vendue par la Société DOW CORNING 3,0 g
- Chlorure de sodium 3,0 g
- pH spontané = 3,7
- Eau qsp 100,0 g

## Revendications

**1.** Composition lavante pour les cheveux ou la peau, caractérisée par le fait qu'elle comporte dans un milieu aqueux :

a) une silicone insoluble dans ledit milieu et non réactive avec celui-ci;

b) au moins 7% en poids d'au moins un composé répondant à la formule suivante :

$$R\text{-}(OC_3H_6)_p\text{-}(OC_2H_4)_n\text{-}OCH_2\text{-}COOA \qquad (I)$$

dans laquelle :

R désigne un radical ou un mélange de radicaux alkyle ou alcényle en $C_8$-$C_{22}$, linéaire ou ramifié, un alkyl($C_8$-$C_9$)phényle, R'CONH-$CH_2$-$CH_2$- avec R' désignant un radical alkyle ou alcényle, linéaire ou ramifié en $C_{11}$-$C_{21}$;

n est un nombre entier ou décimal compris entre 2 et 24;
p est un nombre entier ou décimal compris entre 0 et 6;
A désigne un atome d'hydrogène ou un atome de Na, K, Li, 1/2 Mg ou un reste de monoéthanolamine, ammonium ou triéthanolamine,
le rapport en poids composé de formule (I)/silicone insoluble étant supérieur à 1.

2. Composition selon la revendication 1, caractérisée par le fait que la silicone est choisie parmi les polyorganosiloxanes ne portant pas de groupement ammonium quaternaire, insolubles dans les milieux aqueux et non réactifs avec ces derniers, se présentant sous forme d'huiles, de cires, de gommes ou de résines.

3. Composition selon la revendication 1 ou 2, caractérisée par le fait que la silicone est une silicone volatile ayant un point d'ébullition compris entre 60 et 260°C, ou bien une silicone non volatile choisie parmi les polyalkylsiloxanes, les polyarylsiloxanes, les polyalkylarylsiloxanes, les copolymères de polyéthersiloxanes modifiés ou non, les gommes et résines de silicones, les polysiloxanes organomodifiés, ainsi que leurs mélanges.

4. Composition selon la revendication 3, caractérisée par le fait que la silicone volatile est choisie parmi :
(i) les silicones cycliques de 3 à 7 atomes de silicium, et de préférence 4 à 5, leurs mélanges avec des composés organiques dérivés du silicium, ou bien des cyclocopolymères de formule suivante :

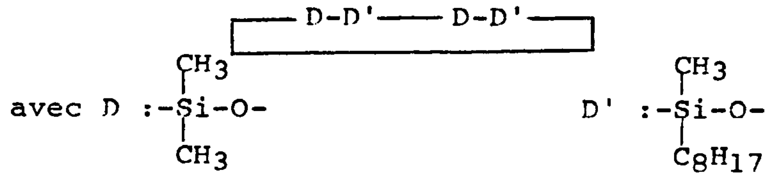
avec

(ii) les silicones linéaires ayant 2 à 9 atomes de silicium et possédant une viscosité inférieure ou égale à $5.10^{-6}$ $m^2/s$ à 25°C.

5. Composition selon la revendication 3, caractérisée par le fait que la silicone non volatile est choisie parmi :
A) les polydiméthylsiloxanes linéaires à groupements terminaux triméthylsilyle, ayant une viscosité de $5.10^{-6}$ à 2,5 $m^2/s$ à 25°C et de préférence $10^{-5}$ à 1 $m^2/s$;
B) les polydiméthylsiloxanes linéaires à groupements terminaux diméthylsilanol;
C) les polyalkyl($C_1$-$C_{20}$)siloxanes;
D) les poly(diméthyl méthyl phényl siloxanes), les polyméthylphénylsiloxanes, les polydiméthyldiphénylsiloxanes linéaires et/ou ramifiés, de viscosité $10^{-5}$ à $5.10^{-2}$ $m^2/s$ à 25°C;
E) les gommes de silicones de masse moléculaire comprises entre 200.000 et 1.000.000, utilisées seules ou sous forme de mélanges dans un solvant, choisies dans le groupe constitué par les copolymères suivants :
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)];

et les mélanges suivants :
- les mélanges formés à partir d'un polydiméthylsiloxane hydroxylé en bout de chaîne et d'un polydiméthylsiloxane cyclique;
- les mélanges formés à partir d'une gomme polydiméthylsiloxane et d'une silicone cyclique;
- les mélanges de deux polydiméthylsiloxanes de viscosités différentes;

F) les résines d'organosiloxanes qui sont des systèmes siloxaniques réticulés, renfermant des unités $R'_2SiO_{2/2}$, $R'SiO_{3/2}$ et $SiO_{4/2}$, dans lesquelles R' représente un groupement hydrocarboné ayant de 1 à 6 atomes de carbone ou un groupement phényle;
G) les silicones organomodifiées choisies parmi les silicones comportant dans leur structure, un ou plusieurs groupements organofonctionnels directement fixés sur la chaîne siloxanique ou fixés par l'intermédiaire d'un radical hydrocarboné.

6. Composition selon la revendication 5, caractérisée par le fait que les silicones organomodifiées sont choisies parmi les polyorganosiloxanes comportant :

a) des groupements polyéthylèneoxy et/ou polypropylèneoxy;
b) des groupements aminés, substitués ou non;
c) des groupements thiols;
d) des groupements carboxylates;
e) des groupements alcoxylés;
f) des groupements hydroxyalkyle, répondant à la formule suivante :

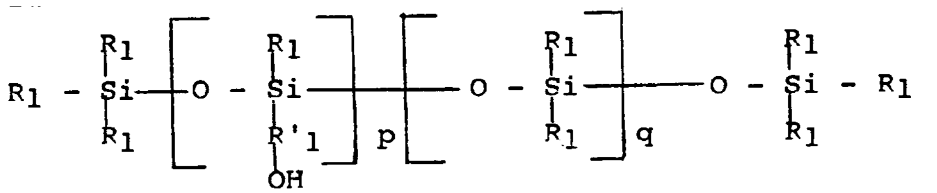

(II)

dans laquelle :

- les radicaux $R_1$, identiques ou différents, sont choisis parmi les radicaux méthyle et phényle, au moins 60% en moles des radicaux $R_1$ étant méthyle;
- le radical $R'_1$ est un chaînon alkylène divalent hydrocarboné en $C_2$-$C_{18}$;
- p est compris entre 1 et 30 inclus;
- q est compris entre 1 et 150 inclus.

g) des groupements acyloxyalkyle, répondant à la formule suivante :

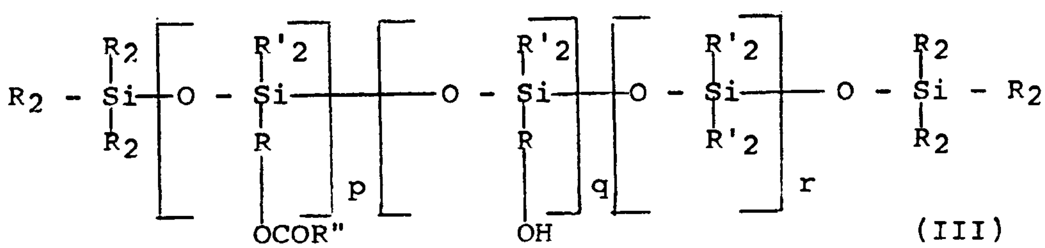

(III)

dans laquelle :

- $R_2$ désigne méthyle, phényle, -OCOR", hydroxyle, un seul des $R_2$ par atome de silicium peut être OH;
- $R'_2$ désigne méthyle, phényle, 60% molaire au moins de l'ensemble des radicaux $R_2$ et $R'_2$ est méthyle;
- R" désigne alkyle ou alcényle en $C_8$-$C_{20}$;
- R désigne un alkylène hydrocarboné divalent, linéaire ou ramifié, en $C_2$-$C_{18}$;
- r est compris entre 1 et 120 inclus;
- p est compris entre 1 et 30;
- q vaut 0 ou est inférieur à 0,5 p, p+q étant compris entre 1 et 30; les polyorganosiloxanes de formule (III) peuvent contenir des groupements

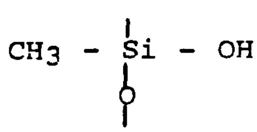

dans des proportions ne dépassant pas 15% de la somme p+q+r;

h) des groupements anioniques de type 2-hydroxyalkylthiosulfate, 2-hydroxyalkylsulfonate ou alkylcarboxylique.

7. Composition selon l'une quelconque des revendications 1 à 6, caractérisée par le fait que le composé de formule (I) est choisi parmi ceux dans lesquels R désigne un radical ou un mélange de radicaux allyle en $C_{12}$-$C_{14}$, oléyle, cétyle ou stéaryle; un radical octylphényle ou nonylphényle, p est égal à 0 et A désigne un atome d'hydrogène ou de sodium.

**8.** Composition selon l'une quelconque des revendications 1 à 7, caractérisée par le fait que la silicone est présente dans des proportions comprises entre 0,2 et 30% et de préférence entre 0,2 et 10% en poids par rapport au poids total de la composition.

**9.** Composition selon l'une quelconque des revendications 1 à 8, caractérisée par le fait que le composé de formule (I) est présent dans des proportions comprises entre 7 et 50% et de préférence entre 8 et 30% en poids par rapport au poids total de la composition.

**10.** Composition selon l'une quelconque des revendications 1 à 9, caractérisée par le fait qu'elle se présente sous la forme d'un shampooing, de crème lavante, d'un gel douche ou de bain moussant.

**11.** Composition selon l'une quelconque des revendications 1 à 10, caractérisée par le fait qu'elle contient en outre un tensio-actif anionique, amphotère, zwittérionique ou non-ionique, ou leurs mélanges.

**12.** Composition selon la revendication 11, caractérisée par le fait que le tensio-actif anionique est choisi parmi :

a) les sels d'ammonium, les sels alcalins, les sels d'amines ou les sels d'aminoalcools des composés suivants :

- les alkylsulfates, alkyléthersulfates, alkylamidesulfates et éthersulfates, alcanolamidesulfates, alkylarylpolyéthersulfates, monoglycéridesulfates,
- les alkylsulfonates, alkylamidesulfonates, alkylarylsulfonates, oléfinesulfonates, paraffinesulfonates,
- les alkylsulfosuccinates, alkyléthersulfosuccinates, alkylamidesulfosuccinates,
- les alkylsulfosuccinamates,
- les alkylsulfoacétates,
- les acylsarcosinates, acylpolypeptidates, acylamidopolypeptidates, acyliséthionates, N-acyltaurates;

les radicaux alkyle et acyle comportant 8 à 18 atomes de carbone;

b) les sels d'acides gras et les acyl($C_8$-$C_{20}$) lactylates.

**13.** Composition selon la revendication 11, caractérisée par le fait que le tensio-actif non ionique est choisi parmi :

a) les alcools, alkylphénols, acides gras polyoxyéthylénés, polypropoxylés ou polyglycérolés à chaîne grasse comportant 8 à 18 atomes de carbone et comprenant 2 à 50 groupements d'oxyde d'éthylène ou d'oxyde de propylène ou 2 à 30 groupements glycérol;

b) les copolymères d'oxyde d'éthylène et de propylène, les condensats d'oxyde d'éthylène et de propylène sur des alcools gras, les amides gras polyoxyéthylénés, les amines grasses polyoxyéthylénées, les éthanolamides, les esters d'acides gras de glycols, les esters d'acides gras du sorbitan oxyéthylénés ou non, les esters d'acides gras du saccharose, les esters d'acides gras de polyéthylèneglycol, les esters d'acides gras de dérivés du glucose, les oxydes d'amines;

c) les composés de formule (IV) :

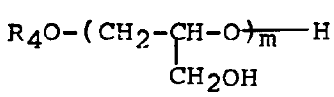

(IV)

dans laquelle $R_4$ désigne un radical ou un mélange de radicaux alkyles contenant 10 à 14 atomes de carbone et m est un nombre entier ou décimal de 2 à 10 et de préférence de 3 à 6;

d) les composés de formule (V) :

$$R_5\text{-CONH-}CH_2\text{-}CH_2\text{-O-}CH_2\text{-}CH_2\text{-O-(}CH_2\text{-CHOH-}CH_2\text{-O)}_nH \quad (V)$$

dans laquelle $R_5$ désigne un radical ou un mélange de radicaux alkyles et/ou alcényles ayant de 11 à 17 atomes de carbone et n désigne un nombre entier ou décimal de 1 à 5, et de préférence 1,5 à 4;

e) les composés de formule (VI) :

$$R_6\text{-CHOH-}CH_2\text{-O-(}CH_2\text{-CHOH-}CH_2\text{-O)}_p\text{-H} \quad (VI)$$

dans laquelle $R_6$ désigne un radical aliphatique, cycloaliphatique, arylaliphatique, ayant de préférence 7 à 21 atomes de carbone, et leurs mélanges, les chaînes aliphatiques désignant en particulier des chaînes alkyle pouvant comporter 1 à 6 groupements éther, thioéther et/ou hydroxyméthylène et p est compris entre 1 et 10 inclus;

f) les composés obtenus par condensation en catalyse acide de 2 à 10 et de préférence 2,5 à 6 moles de glycidol par mole d'alcool ou d'alpha-diol en $C_{10}$-$C_{14}$;

g) les poly(hydroxypropyl)éthers obtenus par poly-addition de monochlorhydrine du glycérol sur un composé organique (poly)hydroxylé en présence d'une base forte.

**14.** Composition selon la revendication 13, caractérisée par le fait que le tensio-actif non-ionique est choisi parmi :

(α) - les composés répondant aux formules (VII) et (VIII) suivantes :

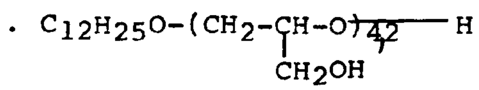 (VII)

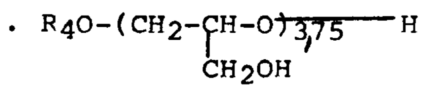 (VIII)

dans laquelle $R_4$ désigne un mélange de radicaux alkyle $C_{10}H_{21}$ et $C_{12}H_{25}$;

(β) - les composés préparés par condensation en catalyse alcaline, de 3,5 moles de glycidol sur un alpha-diol ayant 12 atomes de carbone;

(γ) - les composés répondant à la formule :

$$R_6-CONH-CH_2-CH_2-O-CH_2-CH_2-O-(CH_2-CHOH-CH_2-O)_{3,5}-H \quad (IX)$$

où $R_6$ désigne un mélange de radicaux comprenant les radicaux alkyle et alcényle suivants : $C_{11}H_{23}$, $C_{13}H_{27}$, les radicaux dérivés des acides gras du coprah et le radical dérivé de l'acide oléique;

(δ) - les composés préparés par condensation de 3,5 moles de glycidol sur un mélange d'alpha-diols en $C_{11}$-$C_{14}$;

(ε) - le composé préparé par condensation de 2,5 moles de monochlorhydrine du glycérol sur le dodécanediol-1,2 en présence de soude.

**15.** Composition selon la revendication 11, caractérisée par le fait que le tensio-actif amphotère ou zwittérionique additionnel est choisi parmi :

a) les dérivés d'amines secondaires ou tertiaires aliphatiques, dans lesquels le radical aliphatique est une chaîne linéaire ou ramifiée de 6 à 18 atomes de carbone et contenant au moins un groupe anionique hydrosoluble carboxylique, sulfonate, sulfate, phosphate ou phosphonate;

b) les alkyl($C_{10}$-$C_{20}$)bétaïnes, les sulfobétaïnes, les amidobétaïnes ou les amidosulfobétaïnes.

**16.** Composition selon la revendication 15, caractérisée par le fait que les tensio-actifs amphotères sont choisis parmi les composés de formule :

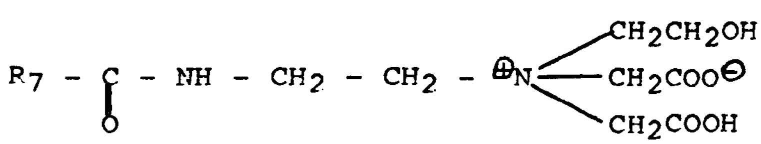

dans laquelle $R_7$ désigne un radical alkyle dérivé du coprah, un radical heptyle, nonyle ou undécyle;

ou les composés de formule :

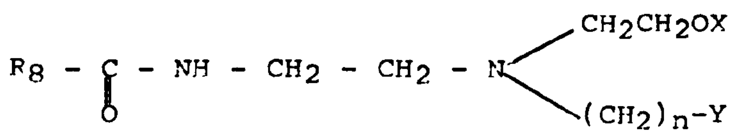

dans laquelle :
n = 1 ou 2;
X désigne le groupement $-CH_2CH_2COOH$ ou hydrogène;
Y désigne -COOH ou le radical

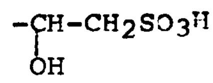

$R_8$ désigne un radical alkyle dérivé du coprah, un radical alkyle en $C_7$, $C_9$, $C_{11}$, $C_{13}$, un radical alkyle en $C_{17}$ et sa forme iso, un radical en $C_{17}$ insaturé, un radical alkyle dérivé de l'huile de lin.

**17.** Composition selon les revendications 11, 12, 15, 16, caractérisée par le fait qu'elle contient un mélange d'agents tensio-actifs anioniques et d'agents tensio-actifs amphotères, zwittérioniques qui représentent jusqu'à 50% et de préférence 5 à 30% en poids du poids total des agents tensio-actifs.

**18.** Composition selon les revendications 11 à 14, caractérisée par le fait qu'elle contient un mélange d'agents tensio-actifs anioniques et d'agents tensio-actifs non-ioniques qui représentent jusqu'à 80% et de préférence 5 à 50% du poids total des agents tensio-actifs.

**19.** Composition selon l'une quelconque des revendications 1 à 18, caractérisée par le fait qu'elle se présente sous la forme d'un produit limpide et que le composé de formule (I) est choisi parmi ceux pour lesquels :
- A désigne hydrogène, R désigne nonylphényle, n vaut 7 et p est égal à 0,
  et des mélanges de composés de formule (I), pour lesquels :
- A désigne hydrogène, R désigne lauryle, n vaut 10 et p est égal à 0; R désigne octylphényle, n vaut 4 ou 8, p est égal à 0 et A désigne hydrogène.

**20.** Composition selon l'une quelconque des revendications 1 à 18, caractérisée par le fait qu'elle se présente sous la forme d'un produit opaque et que le composé de formule (I) est choisi parmi ceux pour lesquels :
- A désigne hydrogène, R désigne octylphényle, n vaut 4 et p vaut 0;
- A désigne hydrogène, R désigne nonylphényle, n vaut 4 et p vaut 0;
- A désigne hydrogène, R désigne oléyle, n vaut 2 ou 5 et p vaut 0;
- A désigne hydrogène, R désigne alkyle en $C_{12}$-$C_{14}$, n vaut 2,5 et p vaut 0.

**21.** Composition limpide selon la revendication 19, caractérisée par le fait que la silicone est choisie parmi :
- les silicones volatiles cycliques de 3 à 7 atomes de silicium, ou leurs mélanges avec des composés organosiliciés ;
- les huiles PDMS linéaires de 2 à 9 atomes de silicium, ayant une viscosité inférieure à $2.10^{-6}$ $m^2/s$;
- les huiles PPMS de viscosité inférieure à $5.\ 10^{-5}$ $m^2/s$.

**22.** Composition opaque selon la revendication 20, caractérisée par le fait que la silicone est choisie parmi :
- les huiles de silicone de viscosité comprise entre 0,2 et 2,5 $m^2/s$ à 25°C;
- les mélanges d'organopolysiloxanes et de silicones cycliques;
- les mélanges de deux polydiméthylsiloxanes de viscosités différentes;
- les organopolysiloxanes modifiés de formules (II) ou (III), telles que définies dans la revendication 6.

**23.** Composition selon l'une quelconque des revendications 11 à 22, caractérisée par le fait que la concentration en tensio-actif additionnel ne dépasse pas 40% du poids total de la composition.

**24.** Composition selon l'une quelconque des revendications 1 à 23, caractérisée par le fait qu'elle présente un pH compris entre 2 et 9.

**25.** Composition selon l'une quelconque des revendications 1 à 24, caractérisée par le fait qu'elle se présente sous forme de liquide plus ou moins épaissi, de gel ou de mousse aérosol.

**26.** Composition selon l'une quelconque des revendications 1 à 25, caractérisée par le fait qu'elle contient en plus un agent régulateur de viscosité présent dans une concentration pouvant aller jusqu'à 15% en poids et de préférence jusqu'à 6% en poids par rapport au poids total de la composition.

**27.** Composition selon l'une quelconque des revendications 1 à 26, caractérisée par le fait qu'elle contient en plus des agents de conditionnement des cheveux ou de la peau choisis parmi les agents tensio-actifs cationiques, les polymères anioniques ou cationiques ou amphotères, les protéines quaternisées ou non et les silicones hydrosolubles, qui n'altèrent pas la stabilité de la composition.

**28.** Composition selon la revendication 27, caractérisée par le fait que les silicones hydrosolubles sont présentes dans des proportions allant jusqu'à 10% et de préférence comprises entre 0,5 et 6% en poids par rapport au poids total de la composition.

**29.** Composition selon la revendication 27, caractérisée par le fait que les polymères cationiques ou anioniques, ou amphotères, les agents cationiques ou les protéines quaternisées ou non, sont présents dans des proportions comprises entre 0,01 et 6% en poids par rapport au poids total de la composition.

**30.** Composition selon l'une quelconque des revendications 1 à 29, caractérisée par le fait qu'elle contient en plus, des adjuvants choisis parmi les agents séquestrants, les parfums, les colorants, les conservateurs, les stabilisateurs de mousse, les agents propulseurs, les agents alcalinisants ou acidifiants ou d'autres adjuvants habituellement utilisés en cosmétique.

**31.** Procédé de lavage et de conditionnement des cheveux ou de la peau, caractérisé par le fait que l'on applique sur ceux-ci une composition telle que définie selon l'une quelconque des revendications 1 à 30, cette application étant suivie d'un rinçage.

**Patentansprüche**

**1.** Waschzusammensetzung für die Haare oder die Haut,
dadurch **gekennzeichnet,** daß
sie in einem wässrigen Medium umfaßt:
a) ein Silikon, das im letztgenannten Medium unlöslich und mit diesem nicht reaktiv ist;
b) mindestens 7 Gew.% mindestens einer Verbindung der folgenden Formel:

$$R\text{- }(OC_3H_6)_p\text{-}(OC_2H_4)_n\text{-}OCH_2COOA \quad (I)$$

worin:
R einen linearen oder verzweigten $C_{8-22}$-Alkyl- oder -Alkenylrest oder eine Mischung dieser Reste, einen $C_{8-9}$-Alkylphenylrest oder einen R'CONH-$CH_2$-$CH_2$-Rest darstellt, wobei R' einen linearen oder verzweigten $C_{11-21}$-Alkyl- oder -Alkenylrest darstellt,
n eine ganze Zahl oder Dezimalzahl von 2 bis 24 ist,
p eine ganze Zahl oder Dezimalzahl von 0 bis 6 ist, und
A ein Wasserstoffatom oder auch Na, K, Li, 1/2 Mg oder einen Monoethanolamin-, Ammonium- oder Triethanolaminrest bedeutet, wobei das Gewichtsverhältnis aus Verbindung der Formel (I)/unlösliches Silikon größer als 1 ist.

**2.** Zusammensetzung gemäß Anspruch 1,
dadurch **gekennzeichnet,** daß
das Silikon aus Polyorganosiloxanen ausgewählt ist, die keine quaternären Ammoniumgruppen aufweisen, im wässrigen Medium unlöslich und damit nicht reaktiv sind und in Form von Ölen, Wachsen, gummiartigen Produkten oder Harzen vorliegen.

**3.** Zusammensetzung gemäß Anspruch 1 oder 2,
dadurch **gekennzeichnet,** daß
das Silikon ein flüchtiges Silikon mit einem Siedepunkt von 60 bis 260°C oder auch ein nicht-flüchtiges Silikon ist, ausgewählt aus Polyalkylsiloxanen, Polyarylsiloxanen, Polyalkylarylsiloxanen, modifizierten oder nicht modifizierten Copolymeren von Polyethersiloxanen, Gummiprodukten und Harzen von Siliko-

nen, organomodifizierten Siloxanen sowie deren Mischungen.

**4.** Zusammensetzung gemäß Anspruch 3,
dadurch **gekennzeichnet,** daß
das flüchtige Silikon ausgewählt ist aus:

(i) zyklischen Silikonen mit 3 bis 7, vorzugsweise 4 bis 5, Siliziumatomen, deren Mischungen mit organischen Siliziumderivatverbindungen, oder auch Cyclocopolymeren der folgenden Formel:

mit 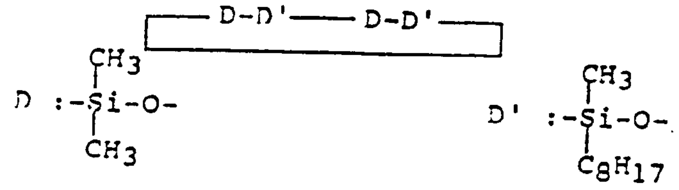

(ii) linearen Silikonen mit 2 bis 9 Siliziumatomen und einer Viskosität von weniger oder gleich 5 x $10^{-6}$ $m^2/s$ bei 25°C.

**5.** Zusammensetzung gemäß Anspruch 3,
dadurch **gekennzeichnet,** daß
das nicht-flüchtige Silikon ausgewählt ist aus:

A) linearen Polydimethylsiloxanen mit endständigen Trimethylsilylgruppen mit einer Viskosität von 5 x $10^{-6}$ bis 2,5 $m^2/s$ bei 25°C und vorzugsweise von $10^{-5}$ bis 1 $m^2/s$;

B) linearen Polydimethylsiloxanen mit endständigen Dimethylsilanolgruppen;

C) Poly($C_{1-20}$)alkylsiloxanen;

D) linearen und/oder verzweigten Poly(dimethylmethylphenylsiloxanen), Polymethylphenylsiloxanen, Polydimethyldiphenylsiloxanen einer Viskosität von $10^{-5}$ bis 5 x $10^{-2}$ $m^2/s$ bei 25°C;

E) Silikon-Gummiprodukten eines Molekulargewichts von 200000 bis 1000000, die alleine oder in Form von Mischungen in einem Losungsmittel vewendet werden, ausgewählt aus der Gruppe aus den folgenden Copolymeren:

- Poly[(dimethylsiloxan)/(methylvinylsiloxan)],
- Poly[(dimethylsiloxan)/(diphenylsiloxan)],
- Poly[(dimethylsiloxan)/(phenylmethylsiloxan)],
- Poly[(dimethylsiloxan)/(diphenylsiloxan)/(methylvinylsiloxan)],
  und den folgenden Mischungen:
- Mischungen, gebildet aus einem am Kettenende hydroxylierten Dimethylsiloxan und einem zyklischen Polydimethylsiloxan;
- Mischungen, gebildet aus einem Polydimethylsiloxan-Gummi und einem zyklischen Silikon;
- Mischungen aus 2 Polydimethylsiloxanen unterschiedlicher Viskositäten;

F) Organosiloxanharzen, die vernetzte Siloxansysteme darstellen, enthaltend Einheiten $R'_2SiO_{2/2}$, $R'SiO_{3/2}$ und $SiO_{4/2}$, in denen R' eine Kohlenwasserstoffgruppe mit 1 bis 6 Kohlenstoffatomen oder eine Phenylgruppe darstellt;

G) organomodifizierten Silikonen, ausgewählt aus Silikonen, enthaltend in ihrer Struktur eine oder mehrere organofunktionelle Gruppen, die an die Siloxankette direkt oder über das Zwischenglied eines Kohlenwasserstoffrestes gebunden sind.

**6.** Zusammensetzung gemäß Anspruch 5,
dadurch **gekennzeichnet,** daß
die organomodifizierten Silikone ausgewählt sind aus Polyorganosiloxanen, enthaltend:

a) Polyethylenoxy- und/oder Polypropylenoxygruppen;
b) substituierte oder nicht substituierte Amingruppen;
c) Thiolgruppen;
d) Carboxylatgruppen;
e) Alkoxygruppen;
f) Hydroxyalkylgruppen der folgenden Formel:

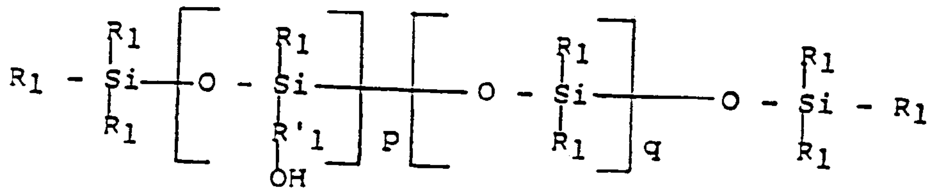

(II)

worin:

- die Reste $R_1$, gleich oder verschieden, ausgewählt sind aus Methyl- und Phenylresten, wobei mindestens 60 Mol% der Reste $R_1$ Methylreste sind;
- der Rest $R'_1$ eine zweiwertige $C_{2-18}$-Alkylenkohlenwasserstoffkette ist;
- p 1 bis 30 beträgt;
- q 1 bis 150 beträgt;

g) Acyloxyalkylgruppen der folgenden Formel:

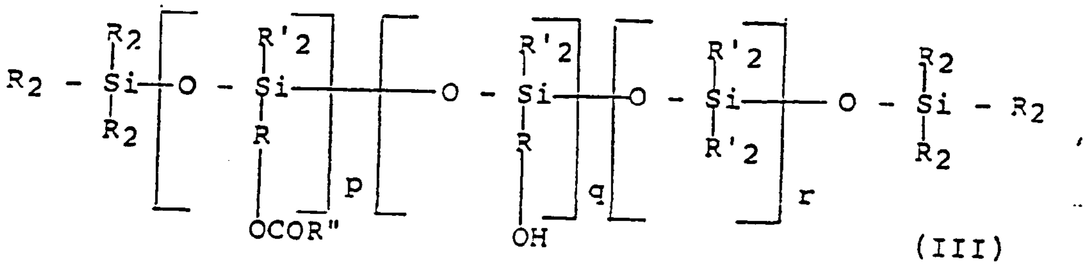

(III)

worin:

- $R_2$ einen Methyl-, Phenyl-, -OCOR"-, Hydroxylrest bedeutet, wobei ein einziger der Reste $R_2$ pro Siliziumatom OH sein kann;
- $R'_2$ einen Methyl-, Phenylrest bedeutet, wobei mindestens 60 Mol% aller Reste $R_2$ und $R'_2$ Methylreste sind;
- R" eine $C_{8-20}$-Alkyl- oder -Alkenylgruppe bedeutet;
- R einen zweiwertigen, linearen oder verzweigten $C_{2-18}$-Alkylenkohlenwasserstoffrest darstellt;
- r 1 bis 120 beträgt;
- p 1 bis 30 beträgt;
- q 0 oder weniger als 0,5p ist, wobei p+q 1 bis 30 beträgt, wobei die Polyorganosiloxane der Formel (III) Gruppen

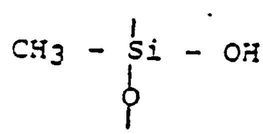

in Mengenverhältnissen enthalten können, die 15% der Summe p+q+r nicht übersteigen;

h) anionische Gruppen vom Typ 2-Hydroxyalkylthiosulfat, 2-Hydroxyalkylsulfonat oder Alkylcarboxylat.

7. Zusammensetzung gemäß jedem Anspruch 1 bis 6,
dadurch **gekennzeichnet,** daß
die Verbindung der Formel (I) unter denjenigen ausgewählt ist, in denen R einen $C_{12-14}$-Alkyl-, Oleyl-, Cetyl- oder Stearylrest, einen Octylphenyl- oder Nonylphenylrest oder eine Mischung dieser Reste darstellt, wobei p gleich 0 ist und A ein Wasserstoff- oder Natriumatom bezeichnet.

8. Zusammensetzung gemäß jedem Anspruch 1 bis 7,
dadurch **gekennzeichnet,** daß
das Silikon in Mengenverhältnissen von 0,2 bis 30, vorzugsweise 0,2 bis 10, Gew.% vorhanden ist, bezogen auf Gesamtgewicht der Zusammensetzung.

**9.** Zusammensetzung gemäß jedem Anspruch 1 bis 8,
dadurch **gekennzeichnet,** daß
die Verbindung der Formel (I) in Mengenverhältnissen von 7 bis 50, vorzugsweise 8 bis 30, Gew.% vorhanden ist, bezogen auf Gesamtgewicht der Zusammensetzung.

**10.** Zusammensetzung gemäß jedem Anspruch 1 bis 9,
dadurch **gekennzeichnet,** daß
sie in Form eines Shampoos, einer Waschcreme, eines Duschgels oder Schaumbads vorliegt.

**11.** Zusammensetzung gemäß jedem Anspruch 1 bis 10,
dadurch **gekennzeichnet,** daß
sie ausserdem ein anionisches, amphoteres, zwitterionisches oder nicht-ionisches oberflächenaktives Mittel oder deren Mischungen enthält.

**12.** Zusammensetzung gemäß Anspruch 11,
dadurch **gekennzeichnet,** daß
das anionische oberflächenaktive Mittel ausgewählt ist aus:

a) Ammonium-, Alkali-, Amin- oder Aminoalkoholsalzen der folgenden Verbindungen:

- Alkylsulfate, Alkylethersulfate, Alkylamidsulfate und -ethersulfate, Alkanolamidsulfate, Alkylarylpolyethersulfate, Monoglyceridsulfate,
- Alkylsulfonate, Alkylamidsulfonate, Alkylarylsulfonate, Olefinsulfonate, Paraffinsulfonate,
- Alkylsulfosuccinate, Alkylethersulfosuccinate, Alkylamidsulfosuccinate,
- Alkylsulfosuccinamate,
- Alkylsulfoacetate,
- Acylsarcosinate, Acylpolypeptidate, Acylamidopolypeptidate, acylierte Thionate, N-Acyltaurate; wobei der Alkyl- oder Acylrest dieser Verbindungen durch eine Kohlenstoffkette mit 8 bis 18 Kohlenstoffatomen dargestellt ist,

b) Fettsäuresalzen und $C_{8-20}$-Acyllactylaten.

**13.** Zusammensetzung gemäß Anspruch 11,
dadurch **gekennzeichnet,** daß
das nicht-ionische oberflächenaktive Mittel ausgewählt ist aus:

a) polyoxyethylierten, polypropoxylierten oder polyglycerierten Alkoholen, Alkylphenolen und Fettsäuren mit einer Fettkette von 8 bis 18 Kohlenstoffatomen, enthaltend 2 bis 50 Ethylen- oder Propylenoxidgruppen und 2 bis 30 Glyceringruppen;

b) den Copolymeren von Ethylen- und Propylenoxid, Kondensaten von Ethylen- und Propylenoxid mit Fettalkoholen, polyoxethylierten Fettamiden, polyoxethylierten Fettaminen, Ethanolamiden, Fettsäureglycolestern, oxethylierten oder nicht oxethylierten Fettsäuresorbitanestern, Fettsäureestern der Saccharose, Fettsäurepolyethylenglycolestern, Fettsäureestern vom Glucosederivaten, Aminoxiden;

c) Verbindungen der Formel (IV) :

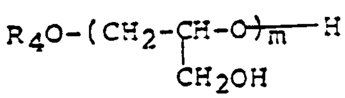

(IV)

worin $R_4$ einen Alkylrest oder eine Mischung von Alkylresten mit 10 bis 14 Kohlenstoffatomen darstellt und m eine ganze Zahl oder Dezimalzahl von 2 bis 10 und vorzugsweise 3 bis 6 ist;

d) Verbindungen der Formel (V) :

$$R_5\text{-CONH-}CH_2\text{-}CH_2\text{-O-}CH_2\text{-}CH_2\text{-O-}(CH_2\text{-CHOH-}CH_2\text{-O})_nH \qquad (V)$$

worin $R_5$ einen Alkyl- und/oder Alkenylrest oder eine Mischung dieser Reste mit 11 bis 17 Kohlenstoffatomen darstellt und n eine ganze Zahl oder Dezimalzahl von 1 bis 5 und vorzugsweise 1,5 bis 4 ist;

e) Verbindungen der Formel (VI) :

$$R_6\text{-CHOH-}CH_2\text{-O-}(CH_2\text{-CHOH-}CH_2\text{-O})_p\text{-H} \qquad (VI)$$

worin $R_6$ einen aliphatischen, cycloaliphatischen, arylaliphatischen Rest mit vorzugsweise 7 bis 21 Kohlenstoffatomen oder deren Mischungen darstellt, wobei die aliphatischen Ketten insbesondere Alkylketten darstellen, die 1 bis 6 Ether-, Thioether- und/oder Hydroxymethylengruppen enthalten können, und worin p 1 bis 10 beträgt;

f) Verbindungen, erhältlich durch Kondensation unter saurer Katalyse von 2 bis 10 und vorzugsweise 2,5 bis 6 Mol Glycidol pro Mol $C_{10-14}$-Alkohol oder -alpha-Diol;

g) Poly(hydroxypropyl)ether, erhältlich durch polyaddition von Glycerinmonochlorhydrin an eine (poly)hydroxylierte organische Verbindung in Gegenwart einer starken Base.

**14.** Zusammensetzung gemäß Anspruch 13,
dadurch **gekennzeichnet,** daß
das nicht-ionische oberflächenaktive Mittel ausgewählt ist aus:
(α) Verbindungen der folgenden Formeln (VII) und (VIII) :

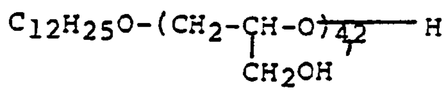
(VII)

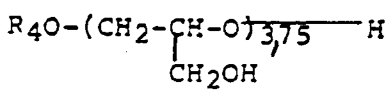
(VIII)

worin $R_4$ eine Mischung von $C_{10}H_{21}$- und $C_{12}H_{25}$-Alkylresten darstellt;
(β) Verbindungen, hergestellt durch Kondensation unter alkalischer Katalyse von 3,5 Mol Glycidol mit einem alpha-Diol mit 12 Kohlenstoffatomen;
(γ) Verbindungen der Formel :

$$R_6-CONH-CH_2-CH_2-O-CH_2-CH_2-O-(CH_2-CHOH-CH_2-O)_{3,5}-H \quad (IX)$$

worin $R_6$ eine Mischung von Resten darstellt, enthaltend die folgenden Alkyl- und Alkenylreste: $C_{11}H_{23}$, $C_{13}H_{27}$, die von Fettsäuren wie Kopra abgeleiteten Reste sowie den von Ölsäure abgeleiteten Rest;
(δ) Verbindungen, hergestellt durch Kondensation von 3,5 Mol Glycidol mit einer Mischung aus $C_{11-14}$-alpha-Diolen;
(ε) - der Verbindung, hergestellt durch Kondensation von 2,5 Mol Glycerinmonochlorhydrin mit Dodecandiol-1,2 in Gegenwart von Soda.

**15.** Zusammensetzung gemäß Anspruch 11,
dadurch **gekennzeichnet,** daß
das amphotere oder zwitterionische zusätzliche oberflächenaktive Mittel ausgewählt ist aus:

a) Derivaten sekundärer oder tertiärer aliphatischer Amine, in denen der aliphatische Rest eine lineare oder verzweigte Kette mit 6 bis 18 Kohlenstoffatomen ist, welcher mindestens eine anionische wasserlösliche Carboxy-, Sulfonat-, Sulfat-, Phosphat- oder Phosphatgruppe enthält;

b) $C_{10-20}$-Alkylbetainen, Sulfobetainen, Amidobetainen oder Amidosulfobetainen.

**16.** Zusammensetzung gemäß Anspruch 15,
dadurch **gekennzeichnet,** daß
die amphoteren oberflächenaktiven Mittel ausgewählt sind aus Verbindungen der Formel:

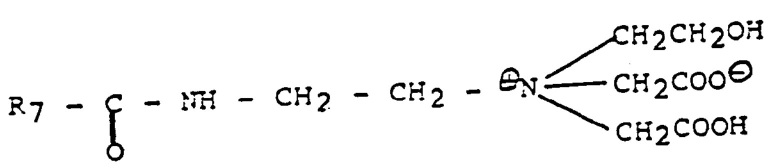

worin $R_7$ einen von Kopra abgeleiteten Alkylrest, einen Heptyl-, Nonyl-, oder Undecylrest darstellt; oder den Verbindungen der Formel :

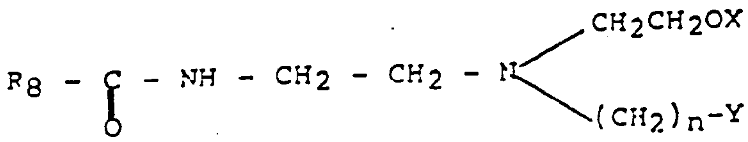

worin n = 1 oder 2;
X bedeutet die Gruppe $-CH_2CH_2COOH$ oder Wasserstoff;
Y bedeutet -COOH oder den Rest

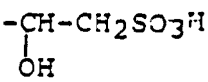

$R_8$ bedeutet einen von Kopra abgeleiteten Alkylrest, einen $C_7$-, $C_9$-, $C_{11}$-, $C_{13}$- Alkylrest, einen $C_{17}$-Alkylrest und seine iso-Form, einen ungesättigten $C_{17}$-Rest, einen von Leinöl abgeleiteten Alkylrest.

**17.** Zusammensetzung gemäß Ansprüchen 11, 12, 15, 16,
dadurch **gekennzeichnet,** daß
sie eine Mischung aus anionischen oberflächenaktiven Mitteln und aus amphoteren, zwitterionischen oberflächenaktiven Mitteln enthält, die bis zu 50 und vorzugsweise von 5 bis 30 Gew.% des Gesamtgewichts der oberflächenaktiven Mittel ausmachen.

**18.** Zusammensetzng gemäß Anspruchen 11 bis 14,
dadurch **gekennzeichnet,** daß
sie eine Mischung aus anionischen oberflächenaktiven Mitteln und aus nicht-ionischen oberflächenaktiven Mitteln enthält, die bis zu 80 und vorzugsweise von 5 bis 50% des Gesamtgewichts der oberflächenaktiven Mittel ausmachen.

**19.** Zusammensetzung gemäß jedem Anspruch 1 bis 18,
dadurch **gekennzeichnet,** daß
sie in Form eines klaren Produkts vorliegt und die Verbindung der Formel (I) aus denjenigen, für die gilt:
- A bedeutet Wasserstoff, R bedeutet Nonylphenyl, n beträgt 7 und p = 0,
  und aus Mischungen von Verbindungen der Formel (I) ausgewählt ist, für die gilt:
- A bedeutet Wasserstoff, R bedeutet Lauryl, n beträgt 10 und p = 0; R bedeutet Octylphenyl, n beträgt 4 oder 8, p=0 und A bedeutet Wasserstoff.

**20.** Zusammensetzung gemäß jedem Anspruch 1 bis 8,
dadurch **gekennzeichnet,** daß
sie in Form eines opaken Produkts vorliegt und die Verbindung der Formel (I) aus denjenigen ausgewählt ist, für die gilt:
- A bedeutet Wasserstoff, R bedeutet Octylphenyl, n beträgt 4 und p beträgt 0;
- A bedeutet Wasserstoff, R bedeutet Nonylphenyl, n beträgt 4 und p beträgt 0;
- A bedeutet Wasserstoff, R bedeutet Oleyl, n beträgt 2 oder 5 und p beträgt 0;
- A bedeutet Wasserstoff, R bedeutet ein $C_{12-14}$-Alkylrest, n beträgt 2,5 und p beträgt 0.

**21.** Klare Zusammensetzung gemäß Anspruch 19,
dadurch **gekennzeichnet,** daß
das Silikon ausgewählt ist aus:
- flüchtigen zyklischen Silikonen mit 3 bis 7 Siliziumatomen oder deren Mischungen mit Organosiliziumverbindungen;
- linearen PDMS-Ölen mit 2 bis 9 Siliziumatomen mit einer Viskosität von weniger als $2 \times 10^{-6}$ $m^2/s$;
- PPMS-Ölen einer Viskosität von weniger als $5 \times 10^{-5}$ $m^2/s$.

**22.** Opake Zusammensetzung gemäß Anspruch 20,
dadurch **gekennzeichnet,** daß

das Silikon ausgewählt ist aus:
- Silikonölen einer Viskosität von 0,2 bis 2,5 $m^2/s$ bei 25°C;
- Mischungen aus Organopolysiloxan und zyklischen Silikonen;
- Mischungen aus 2 Polydimethylsiloxanen unterschiedlicher Viskositäten;
- Modifizierten Organopolysiloxanen der Formeln (II) oder (III), wie definiert in Anspruch 6.

**23.** Zusammensetzung gemäß jedem Anspruch 11 bis 22,
dadurch **gekennzeichnet,** daß
die Konzentration an zusätzlichem oberflächenaktiven Mittel 40% des Gesamtgewichts der Zusammensetzung nicht übersteigt.

**24.** Zusammensetzung gemäß jedem Anspruch 1 bis 23,
dadurch **gekennzeichnet,** daß
sie einen pH von 2 bis 9 aufweist.

**25.** Zusammensetzung gemäß jedem Anspruch 1 bis 24,
dadurch **gekennzeichnet,** daß
sie in Form einer mehr oder weniger verdickten Flüssigkeit, eines Gels oder Aerosolschaums vorliegt.

**26.** Zusammensetzung gemäß jedem Anspruch 1 bis 25,
dadurch **gekennzeichnet,** daß
sie zusätzlich ein Mittel zur Regulierung der Viskosität enthält, das in einer Konzentration vorhanden ist, die bis zu 15 Gew.% und vorzugsweise bis 6 Gew.%, bezogen auf Gesamtgewicht der Zusammensetzung, ausmachen kann.

**27.** Zusammensetzung gemäß jedem Anspruch 1 bis 26,
dadurch **gekennzeichnet,** daß
sie zusätzlich Mittel zum Konditionieren der Haare oder der Haut enthält, ausgewählt aus kationischen oberflächenaktiven Mitteln, anionischen oder kationischen oder amphoteren Polymeren, quaternierten oder nicht quaternierten Proteinen und wasserlöslichen Silikonen, die die Stabilität der Zusammenetzung nicht verändern.

**28.** Zusammensetzung gemäß Anspruch 27,
dadurch **gekennzeichnet,** daß
die wasserlöslichen Silikone in Mengenverhältnissen bis zu 10 und vorzugsweise von 0,5 bis 6 Gew.% vorhanden sind, bezogen auf Gesamtgewicht der Zusammensetzung.

**29.** Zusammensetzung gemäß Anspruch 27,
dadurch **gekennzeichnet,** daß
die kationischen oder anionischen oder amphoteren Polymeren, die oberflächenaktiven kationischen Mittel oder die quaternierten oder nicht quaternierten Proteine in Mengenverhältnissen von 0,01 bis 6 Gew.% vorhanden sind, bezogen auf Gesamtgewicht der Zusammensetzung.

**30.** Zusammensetzung gemäß jedem Anspruch 1 bis 29,
dadurch **gekennzeichnet,** daß
sie zusätzlich Hilfstoffe enthält, ausgewählt aus Sequestrierungsmitteln, Parfüms, Färbemitteln, Konservierungsstoffen, Schaumstabilisiermitteln, Treibmitteln, alkalisch oder sauer machenden Mitteln oder weiteren gewöhnlich in der Kosmetik verwendeten Hilfstoffen.

**31.** Verfahren zum Waschen und Konditionieren der Haare oder der Haut,
dadurch **gekennzeichnet,** daß
man auf diese eine Zusammensetzung gemäß jedem der Ansprüche 1 bis 30 aufbringt, worauf man auf diese Aufbringung eine Spülung folgen läßt.

## Claims

**1.** Washing composition for hair or the skin, comprising in an aqueous medium:
a) a silicone which is insoluble in the said medium and not reactive with the latter;

b) at least 7% by weight of at least one compound corresponding to the following formula:

$$R\text{-}(OC_3H_6)_p\text{-}(OC_2H_4)_n\text{-}OCH_2\text{-}COOA \qquad (I)$$

in which:

R denotes a linear or branched, $C_8$-$C_{22}$ alkyl or alkenyl radical or mixture of such radicals, alkyl($C_8$-$C_9$)-phenyl or R'CONH-$CH_2$-$CH_2$-, with R' denoting a $C_{11}$-$C_{21}$, linear or branched, alkyl or alkenyl radical;

n is a whole or decimal number between 2 and 24,

p is a whole or decimal number between 0 and 6,

A denotes a hydrogen atom or an atom of Na, K, Li, ½ Mg or a monoethanolamine, ammonium or triethanolamine residue,

the weight ratio of compound of formula (I)/insoluble silicone being higher than 1.

**2.** Composition according to Claim 1, in which the silicone is chosen from polyorganosiloxanes containing no quaternary ammonium group, insoluble in aqueous media and not reactive with the latter, which are in the form of oils, waxes, gums or resins.

**3.** Composition according to Claim 1 or 2, characterised in that the silicone is a volatile silicone which has a boiling point of between 60 and 260°C, or else a nonvolatile silicone chosen from polyalkylsiloxanes, polyarylsiloxanes, polyalkylarylsiloxanes, modified or unmodified polyethersiloxane copolymers, silicone gums and resins, organomodified polysiloxanes, and mixtures thereof.

**4.** Composition according to Claim 3, in which the volatile silicone is chosen from:

(i) cyclic silicones containing from 3 to 7, and preferably 4 to 5, carbon atoms, their mixtures with organic compounds derived from silicon, or else cyclocopolymers of following formula:

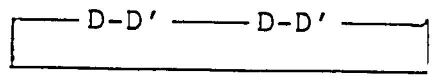

with D : 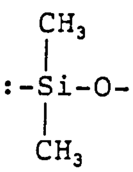 D' : 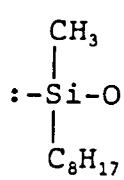

(ii) linear silicones containing 2 to 9 silicon atoms and having a viscosity lower than or equal to $5 \times 10^{-6}$ $m^2/s$ at 25°C.

**5.** Composition according to Claim 3, in which the nonvolatile silicone is chosen from:

A) linear polydimethylsiloxanes containing trimethylsilyl end groups, which have a viscosity of $5 \times 10^{-6}$ to 2.5 $m^2/s$ at 25°C and preferably $10^{-5}$ to 1 $m^2/s$;

B) linear polydimethylsiloxanes containing dimethylsilanol end groups;

C) polyalkyl($C_1$-$C_{20}$)siloxanes;

D) linear and/or branched poly(dimethylmethylphenylsiloxanes), polymethylphenylsiloxanes and polydimethyldiphenylsiloxanes with a viscosity of $10^{-5}$ to $5 \times 10^{-2}$ $m^2/s$ at 25°C;

E) silicone gums of molecular mass of between 200,000 and 1,000,000, employed by themselves or in the form of mixtures in a solvent, chosen from the group consisting of the following copolymers:

- poly[(dimethylsiloxane)/(methylvinylsiloxane)],
- poly[(dimethylsiloxane)/(diphenylsiloxane)],
- poly[(dimethylsiloxane)/(phenylmethylsiloxane)],
- poly[(dimethylsiloxane)/(diphenylsiloxane)/(methylvinylsiloxane)];

and the following mixtures:

- the mixtures made up from a polydimethylsiloxane hydroxylated at a chain end and from a cyclic polydimethylsiloxane;
- the mixtures made up from a polydimethylsiloxane gum and from a cyclic silicone;
- the mixtures of two polydimethylsiloxanes of different viscosities;

F) organosiloxane resins which are crosslinked siloxane systems containing $R'_2SiO_{2/2}$, $R'SiO_{3/2}$ and $SiO_{4/2}$ units, in which R' denotes a hydrocarbon group containing from 1 to 6 carbon atoms or a phenyl

group;

G) organomodified silicones chosen from silicones containing in their structure one or more organofunctional groups attached directly to the siloxane chain or attached by means of a hydrocarbon radical.

**6.** Composition according to Claim 5, in which the organomodified silicones are chosen from polyorganosiloxanes containing

a) polyethyleneoxy and/or polypropyleneoxy groups;
b) amine groups, substituted or otherwise;
c) thiol groups;
d) carboxylate groups;
e) alkoxy groups;
f) hydroxyalkyl groups corresponding to the following formula:

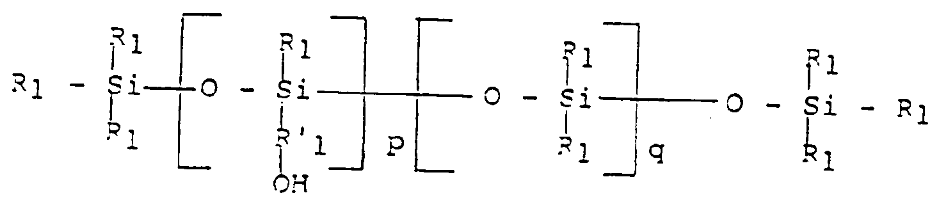
(II)

in which:

- the radicals $R_1$, which are identical or different, are chosen from methyl and phenyl radicals, at least 60 mol% of the radicals $R_1$ being methyl;
- the radical $R'_1$ is a $C_2$-$C_{18}$ divalent alkylene hydrocarbon chain unit;
- p is between 1 and 30 inclusive;
- q is between 1 and 150 inclusive

g) acyloxyalkyl groups corresponding to the following formula:

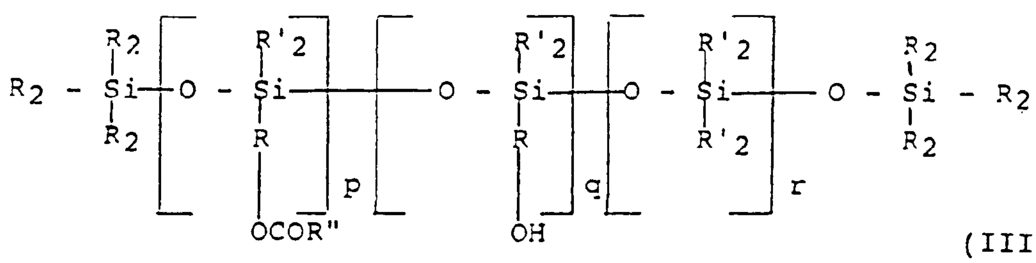
(III)

in which:

- $R_2$ denotes methyl, phenyl, -OCOR" or hydroxyl, only one $R_2$ per silicon atom may be OH;
- $R'_2$ denotes methyl, phenyl, at least 60 mol% of the total of the radicals $R_2$ and $R'_2$ is methyl;
- R" denotes $C_8$-$C_{20}$ alkyl or alkenyl;
- R denotes a $C_2$-$C_{18}$, linear or branched, divalent alkylene hydrocarbon;
- r is between 1 and 120 inclusive;
- p is between 1 and 30;
- q has the value of 0 or is smaller than 0.5 p. p+q being between 1 and 30; the polyorganosiloxanes of formula (III) may contain

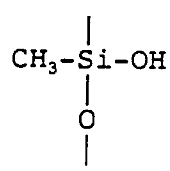

groups in proportions exceeding 15% of the sum p+q+r;

h) anionic groups of the 2-hydroxyalkylthiosulphate, 2-hydroxyalkylsulphonate or alkylcarboxylic type.

7. Composition according to any one of Claims 1 to 6, characterised in that the compound of formula (I) is chosen from those in which R denotes a $C_{12}$-$C_{14}$ alkyl, oleyl, cetyl or stearyl radical or mixture of such radicals, an octylphenyl or nonylphenyl radical, p is equal to 0 and A denotes a hydrogen or sodium atom.

8. Composition according to any one of Claims 1 to 7, characterised in that the silicone is present in proportions of between 0.2 and 30% and preferably between 0.2 and 10%, by weight relative to the total weight of the composition.

9. Composition according to anyone of Claims 1 to 8, characterised in that the compound of formula (I) is present in proportions of between 7 and 50% and preferably between 8 and 30% by weight relative to the total weight of the composition.

10. Composition according to one of Claims 1 to 9, characterised in that it is in the form of a shampoo, washing cream, shower gel or foam bath.

11. Composition according to anyone of Claims 1 to 10, characterised in that it additionally contains an anionic, amphoteric, zwitterionic or nonionic surfactant or mixtures thereof.

12. Composition according to Claim 11, in which the anionic surfactant is chosen from:
a) ammonium salts, alkali metal salts, amine salts or aminoalcohol salts of the following compounds:
- alkylsulphates, alkyl ether sulphates, alkylamide sulphates and ether sulphates, alcanolamide sulphates, alkylaryl polyether sulphates, monoglyceride sulphates,
- alkylsulphonates, alkylamidesulphonates, alkylarylsulphonates, olefinsulphonates, paraffinsulphonates,
- alkylsulphosuccinates, alkyl ethersulphosuccinates, alkylamidesulphosuccinates,
- alkylsulphosuccinamates,
- alkylsulphoacetates,
- acylsarcosinates, acylpolypeptidates, acylamidopolypeptidates, acylisethionates, N-acyltaurates;

the alkyl and acyl radicals containing 8 to 18 carbon atoms;

b) fatty acid salts and acyl($C_8$-$C_{20}$)lactylates.

13. Composition according to Claim 11, characterised in that the nonionic surfactant is chosen from:
a) polyoxyethylenated, polypropoxylated or polyglycerolated alcohols, alkylphenols or fatty acids containing a fatty chain containing 8 to 18 carbon atoms and containing 2 to 50 ethylene oxide or propylene oxide groups or 2 to 30 glycerol groups;
b) copolymers of ethylene and propylene oxide, condensates of ethylene and propylene oxide with fatty alcohols, polyoxyethylenated fatty amides, polyoxyethylenated fatty amines, ethanolamides, glycol fatty acid esters, sorbitan fatty acid esters, oxyethylenated or otherwise, sucrose fatty acid esters, polyethylene glycol fatty acid esters, fatty acid esters of glucose derivatives, amine oxides;
c) the compounds of formula (IV) :

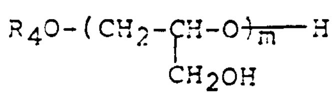

(IV)

in which $R_4$ denotes a radical or a mixture of alkyl radicals containing 10 to 14 carbon atoms and m is a whole or decimal number from 2 to 10 and preferably from 3 to 6;
d) the compounds of formula (V) :

$$R_5\text{-CONH-}CH_2\text{-}CH_2\text{-O-}CH_2\text{-}CH_2\text{-O-(}CH_2\text{-CHOH-}CH_2\text{-O)}_n\text{H} \qquad (V)$$

in which $R_5$ denotes an alkyl and/or alkenyl radical containing from 11 to 17 carbon atoms or a mixture of such radicals and n denotes a whole or decimal number from 1 to 5 and preferably 1.5 to 4;
e) the compounds of formula (VI) :

$$R_6\text{-CHOH-}CH_2\text{-O(}CH_2\text{-CHOH-}CH_2\text{-O)}_p\text{-H} \qquad (VI)$$

in which $R_6$ denotes an aliphatic, cycloaliphatic or arylaliphatic radical preferably containing 7 to 21 carbon atoms, and mixtures thereof, the aliphatic chains denoting in particular alkyl chains which may contain 1 to 6 ether, thioether and/or hydroxymethylene groups, and p is between 1 and 10 inclusive;

f) the compounds obtained by condensation, using acidic catalysis, of 2 to 10 and preferably 2.5 to 6 moles of glycidol per mole of $C_{10}$-$C_{14}$ alcohol or alphadiol;

g) the poly(hydroxypropyl)ethers obtained by polyaddition of glycerol monochlorohydrin to a (poly)hydroxylated organic compound in the presence of a strong base.

**14.** Composition according to Claim 13, in which the nonionic surfactant is chosen from:

(α) - the compounds corresponding to the following formulae (VII) and (VIII) :

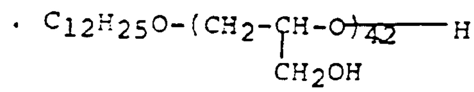

(VII)

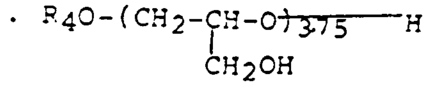

(VIII)

in which $R_4$ denotes a mixture of $C_{10}H_{21}$ and $C_{12}H_{25}$ alkyl radicals;

(β) - the compounds prepared by condensation, using alkaline catalysis, of 3.5 moles of glycidol with an alpha-diol containing 12 carbon atoms;

(γ) the compounds corresponding to the formula:

$$R_6-CONH-CH_2-CH_2-O-CH_2-CH_2-O-(CH_2-CHOH-CH_2-O)_{3.5}-H \quad (IX)$$

where $R_6$ denotes a mixture of radicals containing the following alkyl and alkenyl radicals: $C_{11}H_{23}$, $C_{13}H_{27}$, the radicals derived from copra fatty acids and the radical derived from oleic acid;

(δ) - the compounds prepared by condensing 3.5 moles of glycidol with a mixture of $C_{11}$-$C_{14}$ alpha-diols;

(ε) - the compound prepared by condensing 2.5 moles of glycerol monochlorohydrin with 1,2-dodecanediol in the presence of sodium hydroxide.

**15.** Composition according to Claim 11, in which the additional amphoteric or zwitterionic surfactant is chosen from:

a) derivatives of aliphatic secondary or tertiary amines, in which the aliphatic radical is a linear or branched chain of 6 to 18 carbon atoms and containing at least one water-soluble carboxylic, sulphonate, sulphate, phosphate or phosphonate anionic group;

b) alkyl ($C_{10}$-$C_{20}$)betaines, sulphobetaines, amidobetaines and amidosulphobetaines.

**16.** Composition according to Claim 15, in which the amphoteric surfactants are chosen from the compounds of formula:

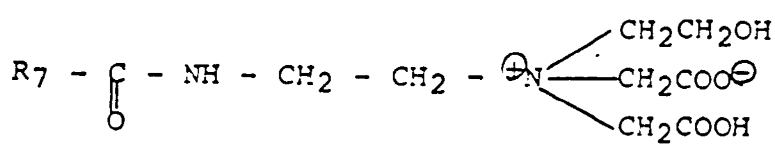

in which $R_7$ denotes an alkyl radical derived from copra, a heptyl, nonyl or undecyl radical;

or the compounds of formula:

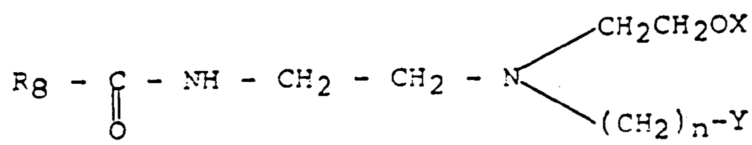

in which:
n = 1 or 2;
X denotes the $-CH_2CH_2COOH$ group or hydrogen;
Y denotes -COOH or the radical

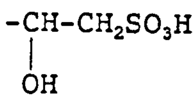

$R_8$ denotes an alkyl radical derived from copra, a $C_7$, $C_9$, $C_{11}$ or $C_{13}$ alkyl radical, a $C_{17}$ alkyl radical and its iso form, an unsaturated $C_{17}$ radical, or an alkyl radical derived from linseed oil.

**17.** Composition according to Claims 11, 12, 15, 16, characterised in that it contains a mixture of anionic surface-active agents and of amphoteric or zwitterionic surface-active agents which represent up to 50% and preferably 5 to 30% total weight of the surface-active agents.

**18.** Composition according to Claims 11 to 14, characterised in that it contains a mixture of anionic surface-active agents and of nonionic surface-active agents which represent up to 80% and preferably 5 to 50% of the total weight of the surface-active agents.

**19.** Composition according to anyone of Claims 1 to 18, characterised in that it is in the form of a clear product and in that the compound of formula (I) is chosen from those in which:
- A denotes hydrogen, R denotes nonylphenyl, n has the value 7 and p is equal to 0, and mixtures of compounds of formula (I), in which:
- A denotes hydrogen, R denotes lauryl, n has the value 10 and p is equal to 0; R denotes octylphenyl, n has the value 4 or 8, p is equal to 0 and A denotes hydrogen.

**20.** Composition according to Claim 1, which is in the form of an opaque product and in which the compound of formula (I) is chosen from those in which:
- A denotes hydrogen, R denotes octylphenyl, n has the value 4 and p has the value 0;
- A denotes hydrogen, R denotes nonylphenyl, n has the value 4 and p has the value 0;
- A denotes hydrogen, R denotes oleyl, n has the value 2 or 5, and p has the value 0;
- A denotes hydrogen, R denotes $C_{12}$-$C_{14}$alkyl, n has the value 2.5 and p has the value 0.

**21.** Clear composition according to Claim 19, characterised in that the silicone is chosen from
- cyclic volatile silicones containing 3 to 7 silicon atoms or their mixtures with organosilicon compounds;
- linear PDMS oils containing 2 to 9 silicon atoms, which have a viscosity lower than $2 \times 10^{-6}$ $m^2/s$;
- PPMS oils with a viscosity lower than $5 \times 10^{-5}$ $m^2/s$.

**22.** Opaque composition according to Claim 20, in which the silicone is chosen from:
- silicone oils with a viscosity of between 0.2 and 2.5 $m^2/s$ at 25°C;
- mixtures of organopolysiloxanes and of cyclic silicones;
- mixtures of two polydimethylsiloxanes of different viscosities;
- modified organopolysiloxanes of formulae (II) or (III), as defined in Claim 6.

**23.** Composition according to any one of Claims 11 to 22, characterised in that the concentration of additional surfactant does not exceed 40% of the total weight of the composition.

**24.** Composition according to any one of Claims 1 to 23, characterised in that it exhibits a pH of between 2 and 9.

**25.** Composition according to any one of Claims 1 to 24, characterised in that it is in the form of more or less thickened liquid, gel or aerosol foam.

**26.** Composition according to any one of Claims 1 to 25, characterised in that it additionally contains a viscosity-regulating agent present in a concentration which may range up to 15% by weight and preferably up to 6% by weight relative to the total weight of the composition.

**27.** Composition according to any one of Claim 1 to 26, characterised in that it additionally contains agents for conditioning hair or the skin, which are chosen from cationic surface-active agents, anionic or cationic or amphoteric polymers, quaternised or unquaternised proteins and water-soluble silicones, which do not alter the stability of the composition.

**28.** Composition according to Claim 27, characterised in that water-soluble silicones are present in proportions ranging up to 10% and preferably between 0.5 and 6% by weight relative to the total weight of the composition.

**29.** Composition according to Claim 27, characterised in that the cationic or anionic or amphoteric polymers, the cationic agents or the quaternised or unquaternised proteins are present in proportions of between 0.01 and 6% by weight relative to the total weight of the composition.

**30.** Composition according to Claim 1, additionally containing adjuvants chosen from sequestering agents, perfumes, colorants, stabilisers, foam stabilisers, propelling agents, alkalifying or acidifying agents or other adjuvants usually employed in cosmetics.

**31.** Process for washing and conditioning hair or the skin, characterised in that a composition as defined according to Claim 1 is applied to these, this application being followed by rinsing.